(19) Europäisches Patentamt
European Patent Office
Office européen des brevets

(11) Veröffentlichungsnummer: **0 433 821 B1**

(12) **EUROPÄISCHE PATENTSCHRIFT**

(45) Veröffentlichungstag der Patentschrift: **28.09.94**

(51) Int. Cl.5: **C09B 62/503**, C07C 317/14, D06P 1/384

(21) Anmeldenummer: **90123644.8**

(22) Anmeldetag: **08.12.90**

(54) **Reaktivfarbstoffe mit einem Anker auf Alkenylsulfonylbasis und Benzylverbindungen als deren Zwischenprodukte.**

(30) Priorität: **19.12.89 DE 3941810**

(43) Veröffentlichungstag der Anmeldung:
**26.06.91 Patentblatt 91/26**

(45) Bekanntmachung des Hinweises auf die Patenterteilung:
**28.09.94 Patentblatt 94/39**

(84) Benannte Vertragsstaaten:
**CH DE FR GB IT LI**

(56) Entgegenhaltungen:
**EP-A- 0 013 765**
**EP-A- 0 307 817**

(73) Patentinhaber: **BASF Aktiengesellschaft**
**Carl-Bosch-Strasse 38**
**D-67063 Ludwigshafen (DE)**

(72) Erfinder: **Marschner, Claus, Dr.**
**Franz-Boegler-Weg 3**
**W-6720 Speyer (DE)**
Erfinder: **Jessen, Joerg L., Dr.**
**Maulbeerstueck 20**
**W-6720 Speyer (DE)**
Erfinder: **Pandl, Klaus, Dr.**
**Schumannstrasse 18**
**W-6700 Ludwigshafen (DE)**
Erfinder: **Patsch, Manfred, Dr.**
**Fritz-Wendel-Strasse 4**
**W-6706 Wachenheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 433 821 B1

## Beschreibung

Die Vorliegende Erfindung betrifft Reaktivfarbstoffe der Formel I

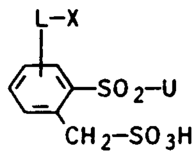 (I),

in der

U Vinyl, Propenyl oder den Rest der Formel

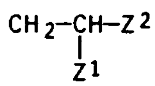 ,

worin $Z^1$ für Wasserstoff oder Methyl und $Z^2$ für eine unter alkalischen Reaktionsbedingungen abspaltbare Gruppe stehen,

X im Fall a) den Rest eines Chromophors, der gegebenenfalls eine weitere reaktive Gruppe aufweist und der sich von einem gegebenenfalls metallisierten Mono- oder Disazofarbstoff, einem Triphendioxazin, einem Anthrachinon, einem Kupfer-Formazan oder einem metallisierten Phthalocyanin ableitet, oder
im Fall b) den Rest einer Kupplungskomponente, an den gegebenenfalls zusätzlich der Rest einer Diazokomponente über eine Azobrücke gebunden ist und der gegebenenfalls eine zusätzliche reaktive Gruppe aufweist, und

L im Fall a) ein Brückenglied der Formel

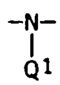 oder 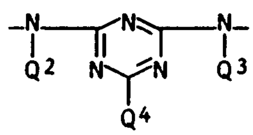 ,

worin $Q^1$ für Wasserstoff oder $C_1$-$C_4$-Alkyl, $Q^2$ und $Q^3$ gleich oder verschieden sind und unabhängig voneinander jeweils für Wasserstoff oder $C_1$-$C_4$-Alkyl und $Q^4$ für eine unter alkalischen Reaktionsbedingungen abspaltbare Gruppe stehen, oder im Fall b) eine Azobrücke bedeuten,
wobei der Rest X-L in meta- oder para-Stellung zum Rest $CH_2$-$SO_3H$ an den Benzolring geknüpft ist,

sowie ein Verfahren zum Färben oder Bedrucken von Hydroxygruppen oder Stickstoff enthaltenden Substraten mittels der neuen Farbstoffe.

Die vorliegende Erfindung betrifft weiterhin neue Benzylverbindungen, die als Zwischenprodukte dieser Farbstoffe dienen.

Gegenstand der EP-A 307 817 sind Doppelankerreaktivfarbstoffe, die sich von gegebenenfalls metallisierten Azofarbstoffen ableiten und ein Doppelankersystem auf Basis eines Triazin/(Sulfonylmethyl)-anilinderivats aufweisen.

Außerdem beschreibt die ältere Patentanmeldung EP-A-352 682 Doppelankerreaktivfarbstoffe, die verschiedenartige Chromophore aufweisen und deren Doppelanker sich von einem Benzolderivat ableitet, das über Alkenylsulfonyl- und Alkenylsulfonylmethylreste verfügt.

Aufgabe der vorliegenden Erfindung war es, neue Reaktivfarbstoffe mit vorteilhaften anwendungstechnischen Eigenschaften bereitzustellen. Die neuen Farbstoffe sollten sich insbesondere für das Kaltverweilverfahren eignen und sollten sich vor allem durch hohe Fixierausbeuten und hohe Faser-Farbstoff-Bindungsstabilitäten auszeichnen. Außerdem sollten die nicht auf der Faser fixierten Anteile leicht auswaschbar sein.

Demgemäß wurden die Reaktivfarbstoffe der obengenannten Formel I gefunden.

Alle in der obengenannten Formel I auftretenden Alkylreste können sowohl geradkettig als auch verzweigt sein.

$Z^2$ und $Q^4$ in Formel I stehen für eine unter alkalischen Reaktionsbedingungen abspaltbare Gruppe. Solche Gruppen sind z.B. Chlor, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino,

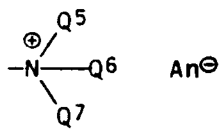 , 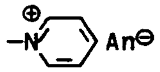 , 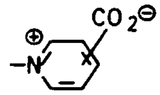 oder 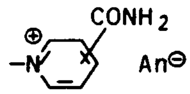 ,

wobei $Q^5$, $Q^6$ und $Q^7$ gleich oder verschieden sind und unabhängig voneinander jeweils die Bedeutung von $C_1$-$C_4$-Alkyl oder Benzyl und $An^{\ominus}$ jeweils die Bedeutung eines Anions besitzen. Als Anion $An^{\ominus}$ kann dabei z.B. Fluorid, Chlorid, Bromid, Iodid, Mono-, Di- oder Trichloracetat, Methylsulfonat, Phenylsulfonat oder 2- oder 4-Methylphenylsulfonat in Betracht kommen.

$Q^4$ ist vorzugsweise Fluor, Chlor oder Brom.

Die Reste $Q^1$, $Q^2$, $Q^3$, $Q^5$, $Q^6$ und $Q^7$ stehen beispielsweise für Methyl, Ethyl, Propyl, Isopropyl, Butyl, Isobutyl oder sec-Butyl.

Der faserreaktive Ankerrest der Formel II

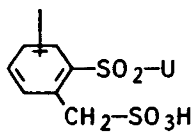 (II),

in der U die obengenannte Bedeutung besitzt, wird im folgenden als "E" bezeichnet.

Bevorzugt sind Reaktivfarbstoffe der Formel Ia

X-L-$E^1$ (Ia),

in der X und L jeweils die obengenannte Bedeutung besitzen und $E^1$ für einen Rest der Formel IIa

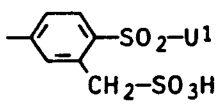 (IIa)

steht, in der

$U^1$ Vinyl oder den Rest der Formel $-CH_2-CH_2-Z^2$ bedeutet, wobei $Z^2$ die obengenannte Bedeutung besitzt.

Neben dem Ankersystem E kann der Rest X noch weitere faserreaktive Reste tragen. Solche Reste leiten sich z.B. von Triazin-, Pyrimidin- oder Vinylsulfonylverbindungen ab.

X in Formel I stellt z.B. den Rest einer Kupplungskomponente dar, an den gegebenenfalls zusätzlich der Rest einer Diazokomponente über eine Azobrücke gebunden ist und der gegebenenfalls eine zusätzliche reaktive Gruppe aufweist. In diesem Fall ist der Doppelankerrest E über eine Azobrücke (-N=N-) an den Rest X geknüpft.

Farbstoffe dieser Klasse gehorchen der Formel IVa oder IVb

$(E-N=N-)_aK$ (IVa)

E-N=N-K-N=N-D (IVb),

wobei K den Rest einer Kupplungskomponente, D den Rest einer Diazokomponente und a 1 oder 2 bedeuten und E die obengenannte Bedeutung besitzt.

Wertvolle Farbstoffe dieser Klasse sind z.B. wasserlösliche Azofarbstoffe, insbesondere Monoazofarbstoffe der Formel IVa (a=1) oder Disazofarbstoffe der Formel IVa (a = 2) oder IVb, die Hydroxysulfonyl- und/oder Carboxylgruppen aufweisen.

Wichtige Kupplungskomponenten HK leiten sich z.B. von Verbindungen aus der Benzol-, Naphthalin-, Pyrazolon-, Pyridon- oder Hydroxypyrimidinreihe ab.

Wichtige Diazokomponenten D-$NH_2$ leiten sich z.B. von Verbindungen aus der Anilin- oder Aminonaphthalinreihe ab.

Besonders bevorzugt sind Farbstoffe der Formel V

$E^1$-N = N-$K^1$ (V),

in der $E^1$ die obengenannte Bedeutung besitzt und $K^1$ für den Rest einer Kupplungskomponente der Naphthalin-, Pyrazol-, Pyridon- oder Hydroxypyrimidinreihe steht, der gegebenenfalls eine weitere faserreaktive Gruppe, insbesondere die Gruppe E, aufweist.

Weiterhin besonders bevorzugt sind Farbstoffe der Formel VI

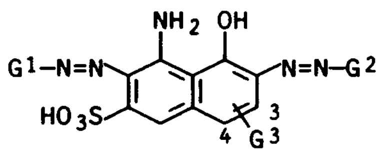

(VI),

in der einer der beiden Reste $G^1$ und $G^2$ für den Rest $E^1$, wobei dieser die obengenannte Bedeutung besitzt, und der andere für den Rest $D^1$, wobei dieser die Bedeutung eines Rest einer Diazokomponente der Anilin- oder Naphthalinreihe, der gegebenenfalls eine weitere faserreaktive Gruppe, insbesondere die Gruppe E, aufweist, besitzt, und $G^3$ für Hydroxysulfonyl in Ringposition 3 oder 4 stehen.

X in Formel I stellt weiterhin z.B. den gegebenenfalls metallisierten Rest eines Azofarbstoffs dar. Geeignete Azofarbstoffe von denen sich solche Reste ableiten, sind an sich bekannt und in großer Zahl beschrieben, z. B. in K. Venkataraman "The Chemistry of Synthetic Dyes", Vol. VI, Academic Press, New York, London, 1972. Die Azofarbstoffe gehorchen der Formel VII

D-N = N-K (-N = N-D)$_l$ (VII),

in der D den Rest einer Diazokomponente, K den Rest einer Kupplungskomponente und l O oder 1 bedeuten.

Wertvolle Farbstoffe, von denen sich der Rest X ableitet, sind z.B. wasserlösliche Azofarbstoffe, insbesondere Monoazofarbstoffe der Formel VII (l = 0), die Hydroxysulfonyl- und/oder Carboxylgruppen aufweisen können.

Bevorzugt leitet sich der Rest X ab von nichtmetallisierten Azofarbstoffen, insbesondere von solchen, die Sulfonsäure- und/oder Carboxylgruppen enthalten, wobei jene, die 1 bis 6 Sulfonsäuregruppen aufweisen, besonders hervorzuheben sind.

Wichtige Azofarbstoffe, von denen sich der Rest X ableitet, sind beispielsweise solche der Phenyl-azo-naphthalin-, Phenyl-azo-1-phenylpyrazol-5-on-,Phenyl-azo-benzol-, Naphthyl-azo-benzol-, Phenyl-azo-aminonaphthalin-,Naphthyl-azo-naphthalin-, Naphthyl-azo-1-phenylpyrazol-5-on-, Phenyl-azo-pyridon-, Phenyl-azo-aminopyridin-, Naphthyl-azo-pyridon-,Naphthyl-azo-aminopyridin- oder Stilbyl-azo-benzolreihe.

Besonders bevorzugt sind Reaktivfarbstoffe der Formel VIII

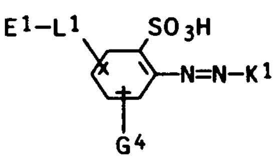

(VIII),

in der $E^1$ die obengenannte Bedeutung besitzt,

$L^1$ den Rest der Formel

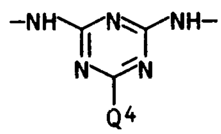

,

worin $Q^4$ die obengenannte Bedeutung besitzt, $G^4$ Wasserstoff, $C_1$-$C_4$-Alkyl, $C_1$-$C_4$-Alkoxy, Chlor, oder Hydroxysulfonyl, und $K^1$ den Rest einer Kupplungskomponente der Naphthalin-, Pyrazolon-, Pyridon- oder Hydroxypyrimidinreihe, der gegebenenfalls eine weitere faserreaktive Gruppe aufweist, bedeuten.

Weiterhin besonders bevorzugt sind Reaktivfarbstoffe der Formel IX

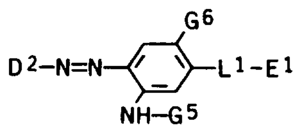

(IX),

in der $E^1$ und $L^1$ jeweils die obengenannte Bedeutung besitzen, $G^5$ für $C_1$-$C_4$-Alkanoyl, Carbamoyl, $C_1$-$C_4$-Mono- oder Dialkylcarbamoyl, Phenylcarbamoyl oder Cyclohexylcarbamoyl, $G^6$ für Wasserstoff, $C_1$-$C_4$-Alkyl, $C_1$-$C_4$-Alkoxy, Hydroxysulfonyl oder Chlor und $D^2$ für den Rest einer Diazokomponente der Anilin- oder Naphthalinreihe, der keine weitere faserreaktive Gruppe trägt, stehen.

Weiterhin besonders bevorzugt sind Reaktivfarbstoffe der Formel X

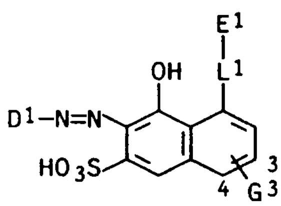

(X),

in der $D^1$, $E^1$ und $L^1$ jeweils die obengenannte Bedeutung besitzen und $G^3$ für Hydroxysulfonyl in Ringposition 3 oder 4 steht.

Weiterhin besonders bevorzugt sind Reaktivfarbstoffe der Formel XI

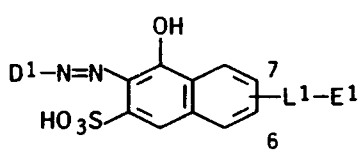

(XI),

in der $D^1$, $E^1$ und $L^1$ jeweils die obengenannte Bedeutung besitzen und die Gruppe -L-$E^1$ in Ringposition 6 oder 7 steht.

Weiterhin sind wertvolle Verbindungen solche der Formel XII

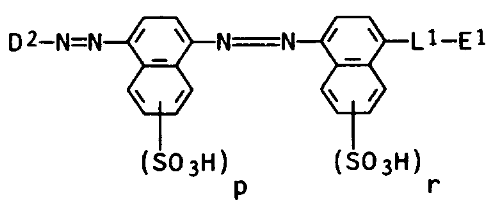

(XII),

in der $D^2$, $E^1$ und $L^1$ jeweils die obengenannte Bedeutung besitzen und p und r unabhängig voneinander jeweils für 0,1 oder 2 stehen.

Weiterhin sind wertvolle Verbindungen solche der Formel XIII

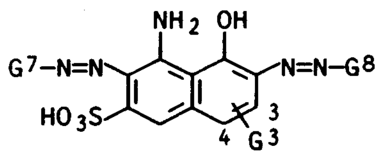

(XIII),

in der $G^3$ die obengenannte Bedeutung besitzt und einer der beiden Reste $G^7$ und $G^8$ für den Rest $D^1$, wobei dieser die obengenannte Bedeutung besitzt,
und der andere für den Rest

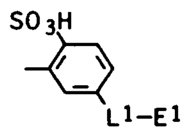

,

in dem $L^1$ und $E^2$ jeweils die obengenannte Bedeutung besitzen, stehen.

Solche aromatischen Reste $D^1$ und $D^2$ von Diazokomponenten der Anilin- oder Aminonaphthalinreihe, die keine faserreaktiven Gruppen tragen, leiten sich beispielsweise von Aminen der Formel XIV a-f

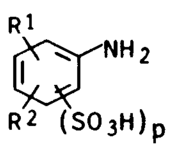

,

(XIVa)

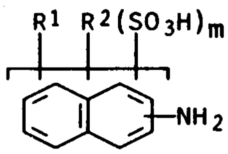

,

(XIVb)

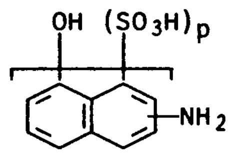

,

(XIVc)

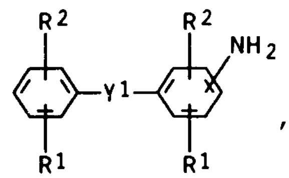

,

(XIVd)

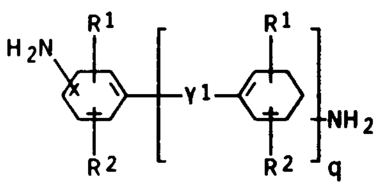

,

(XIVe)

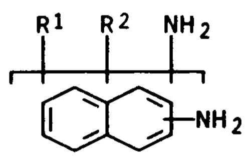

(XIVf)

ab, wobei

- m 0, 1, 2 oder 3,
- P 0, 1 oder 2,
- q 0 oder 1,
- $R^1$ Wasserstoff, Methyl, Ethyl, Methoxy, Ethoxy, Acetyl, Cyano, Carboxyl, Hydroxysulfonyl, $C_1$-$C_4$-Alkoxycarbonyl, Hydroxy, Carbamoyl, $C_1$-$C_4$-Mono- oder Dialkylcarbamoyl, Fluor, Chlor, Brom oder Trifluormethyl,
- $R^2$ Wasserstoff, Methyl, Ethyl, Methoxy, Ethoxy, Cyano, Carboxyl, Hydroxysulfonyl, Acetylamino, $C_1$-$C_4$-Alkoxycarbonyl, Carbamoyl, $C_1$-$C_4$-Mono- oder Dialkylcarbamoyl, Fluor, Chlor, Nitro, Sulfamoyl, $C_1$-$C_4$-Mono- oder Dialkylsulfamoyl, $C_1$-$C_4$-Alkylsulfonyl, Phenylsulfonyl oder Phenoxy und
- $Y^1$ eine direkte Bindung, Sauerstoff, Schwefel oder die Gruppe -NHCO-, -CONH-, -CO-, $-NHSO_2-$, $-SO_2NH-$, $-SO_2-$, -CH = CH-, $-CH_2-CH_2-$, $-CH_2-$, -NH-, oder -N = N- bedeuten.

Bevorzugt sind dabei solche Komponenten, in denen $R^1$ Wasserstoff, Methyl, Methoxy, Carboxyl, Hydroxysulfonyl, Hydroxy oder Chlor, $R^2$ Wasserstoff, Methyl, Methoxy, Carboxyl, Hydroxysulfonyl, Acetylamino oder Chlor und $Y^1$ die Gruppe -CO-, $-SO_2-$, -CH = CH-, $-CH_2-CH_2-$, $-CH_2$-oder -N = N- bedeuten.

Aromatische Amine die sich als Diazokomponenten eignen und die der Formel XIVa, XIVb, XIVc oder XIVd entsprechen, sind beispielsweise Anilin, 2-Methoxyanilin, 2-Methylanilin, 4-Chlor-2-aminoanisol, 4-Methylanilin, 4-Methoxyanilin, 2-Methoxy-5-methylanilin, 2,5-Dimethoxyanilin, 2,5-Dimethylanilin, 2,4-Dimethylanilin, 2,5-Diethoxyanilin, 2-Chloranilin, 3-Chloranilin, 4-Chloranilin, 2,5-Dichloranilin, 4-Chlor-2-nitroanilin, 4-Chlor-2-methylanilin, 3-Chlor-2-methylanilin, 4-Chlor-2-aminotoluol, 4-Phenylsulfonylanilin, 2-Ethoxy-1-naphthylamin, 1-Naphthylamin, 2-Naphthylamin, 4-Methylsulfonylanilin, 2,4-Dichloranilin-5-carbonsäure, 2-Aminobenzoesäure, 4-Aminobenzoesäure, 3-Aminobenzoesäure, 3-Chloranilin-6-carbonsäure, Anilin-2- oder -3- oder -4-sulfonsäure, Anilin-2,5-disulfonsäure, Anilin-2,4-disulfonsäure, Anilin-3,5-disulfonsäure, 2-Aminotoluol-4-sulfonsäure, 2-Aminoanisol-4-sulfonsäure, 2-Aminoanisol-5-sulfonsäure, 2-Ethoxyanilin-5-sulfonsäure, 2-Ethoxyanilin-4-sulfonsäure, 4-Hydroxysulfonyl-2-aminobenzoesäure, 2,5-Dimethoxyanilin-4-sulfonsäure, 2,4-Dimethoxyanilin-5-sulfonsäure, 2-Methoxy-5-methylanilin-4-sulfonsäure, 4-Aminoanisol-3-sulfonsäure, 4-Aminotoluol-3-sulfonsäure, 2-Aminotoluol-5-sulfonsäure, 2-Chloranilin-4-sulfonsäure, 2-Chloranilin-5-sulfonsäure, 2-Bromanilin-4-sulfonsäure, 2,6-Dichloranilin-4-sulfonsäure, 2,6-Dimethylanilin-3-, oder -4-sulfonsäure, 3-Acetylamino-6-sulfonsäure, 4-Acetylamino-2-hydroxysulfonylanilin, 1-Aminonaphthalin-4-sulfonsäure, 1-Aminonaphthalin-3-sulfonsäure, 1-Aminonaphthalin-5-sulfonsäure, 1-Aminonaphthalin-6-sulfonsäure, 1-Aminonaphthalin-7-sulfonsäure, 1-Aminonaphthalin-3,7-disulfonsäure, 1-Aminonaphthalin-3,6,8-trisulfonsäure, 1-Aminonaphthalin-4,6,8-trisulfonsäure, 2-Napththylamin-5-sulfonsäure oder -6- oder -8-sulfonsäure, 2-Aminonaphthalin-3,6,8-trisulfonsäure, 2-Aminonaphthalin-6,8-disulfonsäure, 2-Aminonaphthalin-1,6-disulfonsäure, 2-Aminonaphthalin-1-sulfonsäure, 2-Aminonaphthalin-1,5-disulfonsäure, 2-Aminonapthalin-3,6-disulfonsäure, 2-Aminonaphthalin-4,8-disulfonsäure, 2-Aminophenol-4-sulfonsäure, 2-Aminophenol-5-sulfonsäure, 3-Aminophenol-6-sulfonsäure, 1-Hydroxy-2-aminonaphthalin-5,8-oder -4,6-disulfonsäure, 4-Aminodiphenylamin, 4-Amino-4'-methoxydiphenylamin, 4-Amino-4'-methoxydiphenylamin-3-sulfonsäure, 4-(2'-Methylphenylazo)-2-methylanilin, 4-Aminoazobenzol, 4'-Nitrophenylazo-1-aminonaphthalin, 4-(6'-Hydroxysulfonylnaphthylazo)-1-aminonaphthalin, 4-(2',5'-Dihydroxysulfonylphenylazo)-1-aminonaphthalin, 4'-Amino-3'-methyl-3-nitrobenzophenon, 4-Aminobenzophenon, 4-(4'-Aminophenylazo)-benzolsulfonsäure, 4-(4'-Amino-3'-methoxyphenylazo)-benzolsulfonsäure oder 2-Ethoxy-1-naphthylamin-6-sulfonsäure.

Aromatische Diamine, die sich als Tetrazokomponenten eignen und die der Formel XIVe oder XIVf entsprechen, sind beispielsweise 1,3-Diaminobenzol, 1,3-Diaminobenzol-4-sulfonsäure, 1,4-Diaminobenzol, 1,4-Diaminobenzol-2-sulfonsäure, 1,4-Diamino-2-methylbenzol, 1,4-Diamino-2-methoxybenzol, 1,3-Diamino-4-methylbenzol, 1,3-Diaminobenzol-5-sulfonsäure, 1,3-Diamino5-methylbenzol, 1,6-Diaminonaphthalin-4-sulfonsäure, 2,6-Diaminonaphthalin-4,8-disulfonsäure, 3,3'-Diaminodiphenylsulfon, 4,4'-Diaminodiphenylsulfon, 4,4'-Diaminostilben-2,2'-disulfonsäure, 2,7'Diaminodiphenylsulfon, 2,7'-Diaminodiphenylsulfon-4,5-disulfonsäure, 4,4'-Diaminobenzophenon, 4,4'-Diamino-3,3'-dinitrobenzophenon, 3,3'-Diamino-4,4'-dichlorbenzophenon, 4,4'- oder 3,3'-Diaminodiphenyl, 4,4'-Diamino-3,3'-dichlordiphenyl, 4,4'-Diamino-3,3'-dimethoxy- oder -3,3' -dimethyl- oder -2,2'-dimethyl- oder -2,2'-dichlor- oder -3,3'-diethoxydiphenyl, 4,4'-Diamino-3,3'-dimethyl-6,6'-dinitrodiphenyl, 4,4'-Diaminodiphenyl-2,2'- oder -3,3'-disulfonsäure, 4,4'-Diamino-3,3'-dimethyl- oder -3,3'-dimethoxy oder -2,2'-dimethoxydiphenyl-6,6'-disulfonsäure, 4,4'-Diamino2,2', 5,5'-tetrachlordiphenyl, 4,4'-Diamino-3,3'-dinitrodiphenyl, 4,4'-Diamino-2,2'-dichlor-5,5'-dimethoxydiphenyl, 4,4'-Diaminodiphenyl-2,2'- oder -3,3'-dicarbonsäure, 4,4'-Diamino-3,3'-dimethyldiphenyl-5,5'-disulfonsäure, 4,4'-Diamino-2-nitrodiphenyl, 4,4'-Diamino-3-ethoxy- oder -3-hydroxysulfonyldiphenyl, 4,4'-Diamino-3,3'-dimethyldiphenyl-5-sulfonsäure, 4-4'-Diaminodiphenylmethan, 4,4'-Diamino-3,3'-dimethyldiphenylmethan, 4,4'-Diamino-2,2', 3,3'-tetramethyldiphenylmethan, 4,4'-Diaminodiphenylethan, 4,4'-Diaminostilben oder 4,4'-Diaminodiphenylmethan-3,3'-dicarbonsäure.

Solche aromatischen Reste $D^1$ von Diazokomponenten aus der Anilin- oder Aminonaphthalinreihe, die einen faserreaktiven Rest E tragen können, leiten sich beispielsweise von Aminen der Formel XVa-c

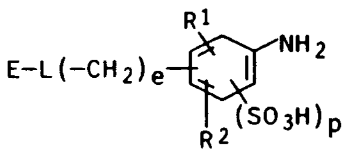 ,
(XVa)

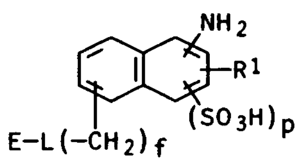 ,
(XVb)

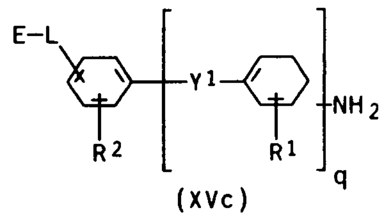
(XVc)

ab, in denen L, $R^1$, $R^2$, p, q, $Y^1$ und E jeweils die obengenannte Bedeutung besitzen und e und f gleich oder verschieden sind und unabhängig voneinander jeweils 0 oder 1 bedeuten.

Aromatische Amine, die den, den faserreaktiven Rest E aufweisenden, Derivaten der Formel XVa, XVb oder XVc zugrundeliegen, sind beispielsweise 1,3-Diaminobenzol,1,3-Diaminobenzol-4-sulfonsäure, 1,3-Diaminobenzol-4,6-disulfonsäure, 1,4-Diaminobenzol, 1,4-Diaminobenzol-2-sulfonsäure, 1,4-Diaminobenzol-2,5-disulfonsäure, 1,4-Diamino-2-methylbenzol , 1,4-Diamino-2-methoxybenzol, 1,3-Diamino-4-methylbenzol, 1,4-Diaminobenzol-2,6-disulfonsäure, 1,5-Diamino-4-methylbenzol-2-sulfonsäure, 1,5-Diamino-4-methoxybenzol-2-sulfonsäure, 1,6-Diaminonapht-2-ol-4-sulfonsäure, 1,6-Diaminonaphthalin-4-sulfonsäure, 2,6-Diaminonaphthalin-4,8-disulfonsäure, 2,6-Diaminonapht-1-ol-4,8-disulfonsäure, 1,3-Diaminobenzol-5-sulfonsäure, 1,3-Diamino-5-methylbenzol, 2,6-Diaminophenol-4-sulfonsäure, 5-(Aminomethyl)-2-aminonaphthalin-1-sulfonsäure, 5-(N-Methylaminomethyl)-2-aminonaphthalin-1-sulfonsäure, 4,4'-Diaminostilben-3,3-dicarbonsäure, 4-(N-Methylaminomethyl)anilin-2-sulfonsäure oder 3-(N-Methylaminomethyl)anilin-6-sulfonsäure.

Die Reste K der Kupplungskomponente entstammen vorzugsweise der Anilin-, Naphthalin-, Pyrazol-, Pyridin-, Pyrimidin-, Indol- oder Acylacetarylidreihe und können auch faserreaktive Gruppen tragen.

Faserreaktivegruppenfreie Kupplungskomponenten der Anilin- oder Naphthalinreihe entsprechen beispielsweise den Verbindungen der Formel XVIa-g

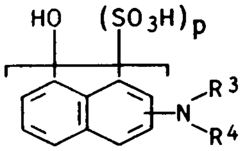
(XVI a)
,
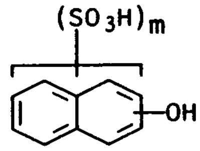
(XVI b)
,
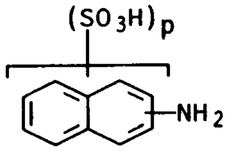
(XVI c)
,
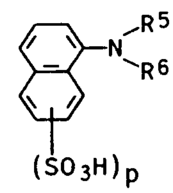
(XVI d)
,
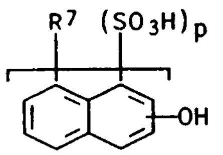
(XVI e)
,
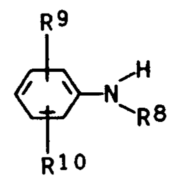
(XVI f)
,
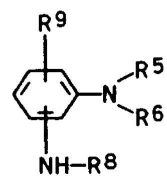
(XVI g)
,

wobei

- $R^3$ Wasserstoff oder $C_1$-$C_4$-Alkyl,
- $R^4$ Wasserstoff, $C_1$-$C_4$-Alkyl oder Phenyl, das durch $C_1$-$C_4$-Alkyl, $C_1$-$C_4$-Alkoxy, Chlor, Brom oder Hydroxysulfonyl ein- oder zweifach substituiert sein kann,
- $R^5$ Wasserstoff oder $C_1$-$C_4$-Alkyl, das durch Hydroxy, Cyano, Carboxyl, Hydroxysulfonyl, Hydroxysulfonyloxy, Methoxycarbonyl, Ethoxycarbonyl oder Acetoxy substituiert sein kann,
- $R^6$ Wasserstoff, $C_1$-$C_4$-Alkyl, das durch Hydroxy, Cyano, Carboxyl, Hydroxysulfonyl, Hydroxysulfonyloxy, Methoxycarbonyl, Ethoxycarbonyl oder Acetoxy substituiert sein kann, Benzyl oder Phenyl, das durch $C_1$-$C_4$-Alkyl, $C_1$-$C_4$-Alkoxy, Chlor oder Hydroxysulfonyl substituiert sein kann,
- $R^7$ $C_1$-$C_6$-Alkylureido, Phenylureido, das durch Chlor, Methyl, Methoxy, Nitro, Hydroxysulfonyl oder Carboxyl substituiert sein kann, $C_1$-$C_6$-Alkanoylamino, Cyclohexanoylamino, Benzoylamino, das durch Chlor, Methyl, Methoxy, Nitro, Hydroxylsulfonyl oder Carboxyl substituiert sein kann, oder Hydroxy,
- $R^8$ Wasserstoff, $C_1$-$C_6$-Alkyl, insbesondere $C_1$-$C_4$-Alkyl, das jeweils durch Phenyl, $C_1$-$C_4$-Alkoxy, Hydroxy, Phenoxy oder $C_1$-$C_4$-Alkanoyloxy substituiert sein kann, $C_5$-$C_7$-Cycloalkyl, Hydroxysulfonylphenyl, $C_1$-$C_4$-Alkanoyl, Carbamoyl, $C_1$-$C_4$-Mono- oder Dialkylcarbamoyl, Phenylcarbamoyl oder Cyclohexylcarbamoyl,
- $R^9$ Methoxy, Ethoxy, Chlor, Brom, Acetylamino, Amino, Ureido, Methylsulfonylamino, Ethylsulfonylamino, Dimethylaminosulfonylamino, Methylamino, Ethylamino, Dimethylamino oder Diethylamino und
- $R^{10}$ Wasserstoff, Methyl, Ethyl, Methoxy, Ethoxy, Chlor oder Brom bedeuten und
- p und m jeweils die obengenannte Bedeutung besitzen.

Im einzelnen sind beispielsweise o- oder m-Toluidin, o- oder m-Anisidin, Kresidin, 2,5-Dimethylanilin, 2,5-Dimethoxyanilin, m-Aminoacetanilid, 3-Amino-4-methoxyacetanilid, 3-Amino-4-methylacetanilid, m-Aminophenylharnstoff, N-Methylanilin, N-Methyl-m-toluidin, N-Ethylanilin, N-Ethyl-m-toluidin, N-(2-Hydroxyethyl)anilin oder N-(2-Hydroxyethyl)-m-toluidin zu nennen.

Naphtholsulfonsäuren sind beispielsweise 1-Naphthol-3-sulfonsäure, 1-Naphthol-4-sulfonsäure, 1-Naphthol-5-sulfonsäure, 1-Naphthol-8-sulfonsäure, 1-Naphthol-3,6-disulfonsäure, 1-Naphthol-3,8-disulfonsäure, 2-Naphthol-5-sulfonsäure, 2-Naphthol-6-sulfonsäure, 2-Naphthol-7-sulfonsäure, 2-Naphthol-8-sulfonsäure, 2-Napthol-3,6-disulfonsäure, 2-Naphthol-6,8-disulfonsäure, 2-Napthol-3,6,8-trisulfonsäure, 1,8-Dihydroxynaphthalin-3,6-disulfonsäure, 2,6-Dihydroxynapthalin-8-sulfonsäure oder 2,8-Dihydroxynaphthalin-6-sulfonsäure.

Weiterhin sind beispielsweise 1-Naphthylamin, N-Phenyl-1-napthylamin, N-Ethyl-1-naphthylamin, N-Phenyl-2-naphthylamin, 1-Naphthol, 2-Naphthol, 1,5-Dihydroxynaphthalin, 1,6-Dihydroxynaphthalin, 1,7-Dihydroxynaphthalin oder 2,7-Dihydroxynaphthalin zu nennen.

Aminonaphthalinsulfonsäuren sind beispielsweise 1-Naphthylamin-6-sulfonsäure, 1-Naphthylamin-7-sulfonsäure, 1-Naphthylamin-8-sulfonsäure, 2-Naphthylamin-3,6-disulfonsäure, 2-Naphthylamin-5,7-disulfonsäure oder 2-Naphthylamin-6,8-disulfonsäure.

Als Aminonaphtholsulfonsäuren sind z. B. 1-Amino-5-hydroxynaphthalin-7-sulfonsäure, 1-Amino-8-hydroxynaphthalin-4-sulfonsäure, 1-Amino-8-hydroxynaphthalin-2,4-disulfonsäure, 1-Amino-8-hydroxynaphthalin-3,6-disulfonsäure, 1-Amino-8-hydroxynaphthalin-4,6-disulfonsäure, 2-Amino-5-hydroxynaphthalin-7-sulfonsäure, 2-Amino-8-hydroxynaphthalin-6-sulfonsäure, 2-Amino-8-hydroxynaphthalin-3,6-disulfonsäure, 2-Amino-5-hydroxynaphthalin-1,7-disulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure, 1-Benzoylamino-8-hydroxynaphthalin-4,6-disulfonsäure, 1-Acetylamino-5-hydroxynaphthalin-7-sulfonsäure, 2-Methylamino-8-hydroxynaphthalin-6-sulfonsäure, 2-Methylamino-8-hydroxynaphthalin-6-sulfonsäure oder 2-(3'-oder 4'-Hydroxysulfonylphenyl)amino-8-hydroxynaphthalin-6-sulfonsäure zu nennen.

Von besonderer Bedeutung sind Kuppiungskomponenten, die Sulfonsäuren- und/oder Carboxylgruppen aufweisen und die in ortho- oder para-Stellung zu einer Hydroxy- und/oder Aminogruppe kuppeln.

Als Beispiele für solche Kupplungskomponenten seien 2-Acetylamino-5-hydroxynaphthalin-7-sulfonsäure, 2-Acetylamino-8-hydroxynaphthalin-6-sulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure oder 1-Benzoylamino-8-hydroxynaphthalin-4,6-disulfonsäure genannt.

Kupplungskomponenten der weiteren Reihen sind beispielsweise: Pyrazolone Aminopyrazole, 2,6-Diaminopyridine, Pyridone, Hydroxy- oder Aminopyrimidine, Indole oder Acetoacetarylide.

Faserreaktivgruppenfreie Kupplungskomponenten dieser Reihe entsprechen dabei beispielsweise der Formel XVIIa-f.

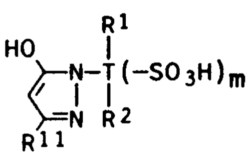
(XVII a)
,
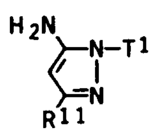
(XVII b)
,
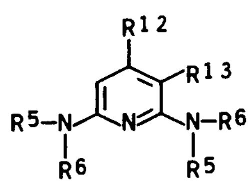
(XVII c)
,
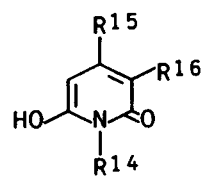
(XVII d)
,
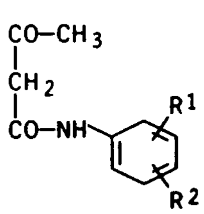
(XVII e)
,
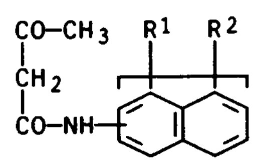
(XVII f)
,

wobei

T für einen Benzol- oder Naphthalinkern,

$T^1$ für $C_1$-$C_4$-Alkyl, Cyclohexyl, Benzyl oder Phenyl, das ein- oder mehrfach durch Fluor, Chlor,

Brom, Methyl, Methoxy, Nitro, Hydroxysulfonyl, Carboxyl, Acetyl, Acetylamino, Methylsulfonyl, Sulfamoyl oder Carbamoyl substituiert ist,

$R^{11}$ für Methyl, Carboxyl, $C_1$-$C_4$-Alkoxycarbonyl oder Phenyl,

$R^{12}$ für Wasserstoff oder $C_1$-$C_4$-Alkyl, das durch Methoxy, Ethoxy oder Cyano substituiert sein kann,

$R^{13}$ für Wasserstoff, Methyl, Hydroxysulfonylmethyl, Hydroxysulfonyl, Cyano oder Carbamoyl,

$R^{14}$ für Wasserstoff, $C_1$-$C_4$-Alkyl, das durch Phenyl, Hydroxysulfonylphenyl, Hydroxy, Amino, Methoxy, Ethoxy, Carboxyl, Hydroxysulfonyl, Acetylamino, Benzoylamino oder Cyano substituiert sein kann, Cyclohexyl, Phenyl, das gegebenenfalls durch Carboxyl, Hydroxysulfonyl, Benzoylamino, Acetylamino, Methyl, Methoxy, Cyano oder Chlor substituiert ist, oder Amino, das durch Phenyl, $C_1$-$C_4$-Alkyl, Acetyl oder Benzoyl substituiert ist,

$R^{15}$ für $C_1$-$C_4$-Alkyl, Phenyl, Hydroxy, Cyano, Acetyl, Benzoyl, Carboxyl, Methoxycarbonyl, Carbamoyl oder Hydroxysulfonylmethyl und

$R^{16}$ für Wasserstoff, Chlor, Brom, Acetylamino, Amino, Nitro, Hydroxysulfonyl, Sulfamoyl, Methylsulfonyl, Phenylsulfonyl, Carboxyl, Methoxycarbonyl, Acetyl, Benzoyl, Carbamoyl, Cyano oder Hydroxysulfonylmethyl stehen und $R^1$, $R^2$, $R^5$, $R^6$ und m jeweils die obengenannte Bedeutung besitzen.

Als Pyrazolon-Kupplungskomponenten sind beispielsweise 3-Methyl-, 3-Carboxy- oder 3-($C_1$-$C_4$-Alkoxycarbonyl)pyrazol-5-one zu nennen, die in 1-Stellung Wasserstoff, gegebenenfalls durch Methyl, Ethyl, Fluor, Chlor, Brom, Trifluormethyl, Methoxy, Ethoxy, Cyano, Phenoxy, Phenylsulfonyl, Methylsulfonyl, Hydroxysulfonyl, Acetylamino, Nitro, Hydroxyl, Carboxyl, Carbamoyl oder Sulfamoyl substituiertes Phenyl oder durch Hydroxysulfonyl substituiertes 1-, oder 2-Naphthyl tragen können. Beispielsweise sind 1-Phenyl-, 1-(2'-Chlorphenyl)-, 1-(2'-Methoxyphenyl)-, 1-(2'Methylphenyl)-, 1-(1',5'-Dichlorphenyl)-, 1-(2',6'-Dichlorphenyl)-, 1-(2'-Methyl-6'-chlorphenyl)-, 1-(2'-Methoxy-5'-methylphenyl)-, 1-(2'-Methoxy-5'-hydroxysulfonyl-phenyl)-, 1-(2',5'-Dichlor-4'-hydroxysulfonylphenyl)-, 1-(2',5'-Dihydroxy-sulfonylphenyl)-, 1-(2'-Carboxyphenyl)-, 1-(3'-Hydroxysulfonylphenyl)-, 1-(4'-Hydroxysulfonylphenyl)- oder 1-(3'-Sulfamoylphenyl)-3-carboxyl-pyrazol-5-on, 1-(3'- oder 4'-Hydroxysulfonylphenyl)-, 1-(2'-Chlor-4'- oder -5'-hydroxysulfonylphenyl)-, 1-(2'-Methyl-4'-hydroxy-sulfonylphenyl)-, 1-(2',5'-Dichlorphenyl)-,1-(4',8'-Dihydroxysulfonyl-1-naphthyl)-, 1-(6'-Hydroxysulfonyl-1-naphthyl)-3-methylpyrazol-5-on, 1-Phenylpyrazol-5-on-3-carbonsäureethylester, Pyrazol-5-on-3-carbonsäure-ethylester oder Pyrazol-5-on-3-carbonsäure zu nennen.

Andere aus der Pyrazolreihe stammende Kupplungskomponenten sind beispielsweise 1-Methyl-, 1-Ethyl-, 1-Propyl-, 1-Butyl-, 1-Cyclohexyl-, 1-Benzyl- oder 1-Phenyl-5-aminopyrazol, 1-(4'-Chlorphenyl)-, 1-(4'-Methylphenyl)-5-aminopyrazol oder 1-Phenyl-3-methyl-5-aminopyrazol.

Acetoacetanilide sind vor allem Acetessiganilid oder dessen im Phenylkern durch Chlor, Methyl, Ethyl, Methoxy, Ethoxy, Acetylamino, Hydroxysulfonyl, Carboxyl, Carbamoyl oder Sulfamoyl ein- oder mehrfach substituierte Derivate.

Vom Pyridin abgeleitete Kupplungskomponenten sind beispielsweise die in der DE-A-2 260 827 beschriebenen Derivate.

Als Pyrimidinkupplungskomponenten sind z. B. die in der DE-A-2 202 820, DE-A-2 308 663 oder DE-A-3 119 349 aufgeführten Verbindungen geeignet. Weiterhin sind Barbitursäure und deren N-Substitutionsprodukte zu nennen. Als N-Substituenten kommen dabei insbesondere $C_1$-$C_4$-Alkyl oder gegebenenfalls substituiertes Phenyl in Betracht.

Als Indolkupplungskomponenten sind beispielsweise 2-Methylindol, 2-Phenylindol, 2-Phenylindol-5-sulfonsäure, 1-Methyl-2-phenylindol, 1-(2'-Hydroxyethyl)-, 1-(2'-Carboxyethyl)-, 1-(2'Carbamoylethyl)-2-methylindol oder -2-phenylindol zu nennen.

Als Pyridonkupplungskomponenten sind beispielsweise 1-Ethyl-2-hydroxy-4-methyl-5-carbamoylpyrid-6-on, 1-(2'Hydroxyethyl)-2-hydroxy-4-methyl-5-carbamoylpyrid-6-on, 1-Phenyl-2-hydroxy-4-methyl-5-carbamoylpyrid-6-on, 1-Ethyl-2-hydroxy-4-methyl-5-cyanopyrid-6-on, 1-Ethyl-2-hydroxy-4-methyl-5-hydroxysulfonylmethylpyrid-6-on, 1-Methyl-2-hydroxy-4-methyl-5-cyanopyrid-6-on, 1-Methyl-2-hydroxy-5-acetylpyrid-6-on, 1,4-Dimethyl-2-hydroxy-5-cyanopyrid-6-on, 1,4-Dimethyl-5-carbamoylpyrid-6-on, 2,6-Dihydroxy-4-ethyl-5-cyanopyridin, 2,6-Dihydroxy-4-ethyl-5-carbamoylpyridin, 1-Ethyl-2-hydroxy-4-methyl-5-hydroxy-sulfonylmethylpyrid-6-on, 1-Methyl-2-hydroxy-4-methyl-5-methyl-sulfonylpyrid-6-on oder 1-Carboxymethyl-2-hydroxy-4-ethyl-5-phenyl-sulfonylpyrid-6-on zu nennen.

Faserreaktivgruppenhaltige Kupplungskomponenten K der Anilin- oder Naphthalinreihe sind beispielsweise Verbindungen der Formel XVIII a - e.

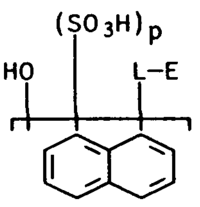 , 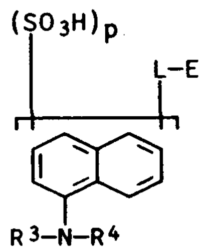 , 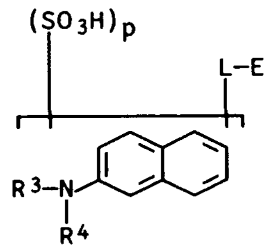 , 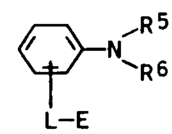 ,

(XVIII a) (XVIII b) (XVIII c) (XVIII d)

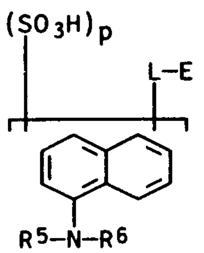 ,

(XVIII e)

wobei L, $R^3$, $R^4$, $R^5$, $R^6$, E und p jeweils die obengenannte Bedeutung besitzen.

Faserreaktivgruppenhaltige Kupplungskomponenten der Pyrazolon-, Aminopyrazol-, 2,6-Diaminopyridin-, Pyridon-, Hydroxy- oder Aminopyrimidin-, Indol-, oder Acetoacetarylidreihe entsprechen beispielsweise der Formel XIX a - f.

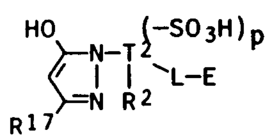 , 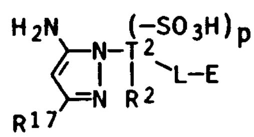 , 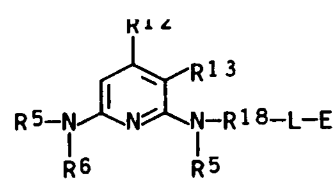 ,

(XIX a) (XIX b) (XIX c)

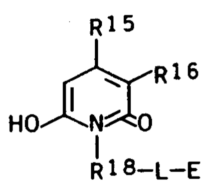 , 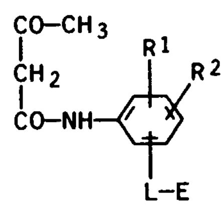 , 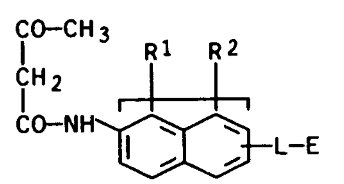 ,

(XIX d) (XIX e) (XIX f)

wobei

| | |
|---|---|
| $T^2$ | für einen Benzol- oder Naphthalinkern |
| $R^{17}$ | für Methyl, Carboxyl, $C_1$-$C_4$-Alkoxycarbonyl oder Phenyl, |
| $R^{18}$ | für $C_1$-$C_4$-Alkyl, Benzyl, Phenylethyl oder Phenyl, wobei die Phenylkerne jeweils noch durch Fluor, Chlor, Brom, Methyl, Methoxy, Cyano, Hydroxysulfonyl, Carboxyl, Acetyl, Nitro, Carbamoyl oder Sulfamoyl substituiert sein können, stehen und |
| L $R^1$, $R^2$, $R^5$, $R^6$, $R^{12}$, $R^{13}$, $R^{15}$, $R^{16}$, p und E | jeweils die obengenannte Bedeutung besitzen. |

Faserreaktive Reste E tragende Pyrazolonkupplungskomponenten leiten sich beispielsweise von folgenden Pyrazolonen ab: 1-(3'- oder 4'-Aminophenyl)-, 1-(2'-Hydroxysulfonyl-5'-aminophenyl)- oder 1-(2'-Methoxy-5'-aminophenyl)-3-carboxylpyrazol-5-on, 1-(3'- oder 4'-Aminophenyl)- oder 1-(6'-Amino4',8'-dihydroxysulfonylnaphth-2'-yl)-3-carboxylpyrazol-5-on.

Anstelle der Azofarbstoffreste können die Farbstoffe der Formel I auch entsprechende Metallkomplexfarbstoffreste enthalten. Als komplexierende Metalle kommen dabei insbesondere Kupfer, Kobalt, Chrom, Nickel oder Eisen in Betracht, wobei Kupfer, Kobalt oder Chrom bevorzugt sind.

Dabei befinden sich die metallisierten Gruppen vorzugsweise jeweils in ortho-Stellung zur Azogruppe, z. B. in Form von o,o'-Dihydroxy-, o-Hydroxy-o'-carboxy-, o-Carboxy-o'-amino- oder o-Hydroxy-o'-aminoazogruppierungen.

X in Formel I stellt weiterhin z.B. den Rest eines Kupfer-Formazanfarbstoffs dar. Kupfer-Formazane sind an sich bekannt und beispielsweise in K. Venkataraman "The Chemistry of Synthetic Dyes", Vol. III, Academic Press, New York, London, 1970, beschrieben.

Besonders bevorzugt sind Kupfer-Formazanfarbstoffe der Formel XX

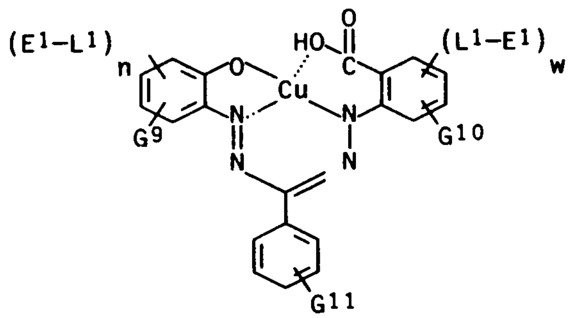

(XX),

in der

| | |
|---|---|
| $G^9$, $G^{10}$ und $G^{11}$ | gleich der verschieden sind und unabhängig voneinander jeweils Wasserstoff oder Hydroxysulfonyl, |
| n | 0 oder 1 und |
| w | 0 oder 1 bedeuten und |
| $E^1$ und $L^1$ | jeweils die obengenannte Bedeutung besitzen, mit der Maßgabe, daß n und w nicht gleichzeitig 0 bedeuten. |

Eine Methode zur Herstellung der diesen Farbstoffen zugrundeliegenden Formazane ist beispielsweise in der EP-A-315 046 beschrieben.

X in Formel I stellt weiterhin z.B. den Rest eines Anthrachinonfarbstoffs dar. Anthrachinone sind an sich bekannt und beispielsweise in K. Venkataraman "The Chemistry of Synthetic Dyes", Vol. II, Academic Press, New York, 1952, beschrieben.

Besonders bevorzugt sind Anthrachinonfarbstoffe der Formel XXI

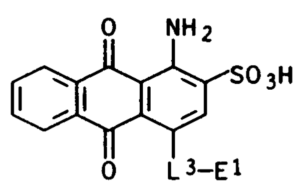

(XXI),

in der

$L^3$ für Imino oder den Rest

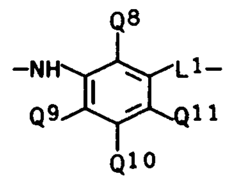

worin $L^1$ die obengenannte Bedeutung besitzt, und $Q^8$ und $Q^9$ gleich oder verschieden sind und unabhängig voneinander jeweils Wasserstoff oder Methyl und einer der beiden Reste $Q^{10}$ und $Q^{11}$ Wasserstoff oder Methyl und der andere Hydroxysulfonyl bedeuten, und $E^1$ die obengenannte Bedeutung besitzt.

X in Formel I stellt weiterhin z.B. den Rest eines Triphendioxazinfarbstoffs dar. Triphendioxazine sind an sich bekannt und beispielsweise in der EP-A-141 359 oder EP-A-311 969 beschrieben.

Besonders bevorzugt sind Triphendioxazinfarbstoffe der Formel XXII

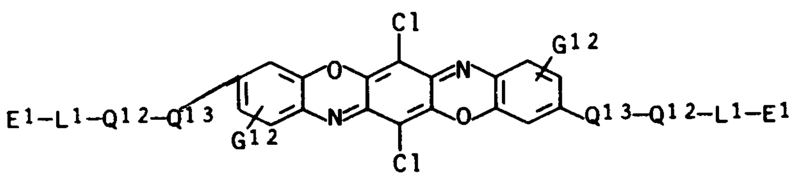

(XXII),

in der

$E^1$ und $L^1$ jeweils die obengenannte Bedeutung besitzen und

$G^{12}$ für Hydroxysulfonyl oder den Rest $SO_2$-$C_2H_4$-$OSO_3H$,

$Q^{13}$ für Sauerstoff, Imino oder $C_1$-$C_4$-Alkylimino und

$Q^{12}$ für geradkettiges oder verzweigtes $C_2$-$C_4$-Alkylen oder Phenylen stehen.

X in der Formel I stellt weiterhin z.B. den Rest eines metallisierten Phthalocyaninfarbstoffs dar. Phthalocyanine sind an sich bekannt und beispielsweise in F.H. Moser, D.L. Thomas "The Phthalocyanines", Vol. II, CRC Press, Boca Raton, Florida, 1983, beschrieben.

Besonders bevorzugt sind Phthalocyaninfarbstoffe der Formel XXIII

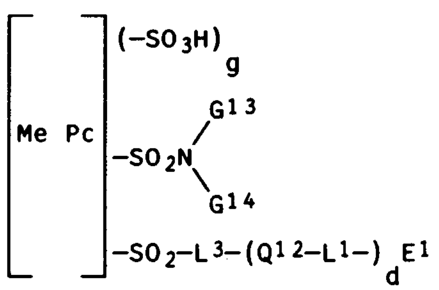

(XXIII),

in der

Pc den Phthalocyaninrest,

$G^{13}$ und $G^{14}$ gleich oder verschieden sind und unabhängig voneinander jeweils Wasserstoff oder $C_1$-$C_4$-Alkyl,

$L^3$ Imino oder $C_1$-$C_4$-Alkylimino,

d 0, 1, 2 oder 3 und

Me Kupfer oder Nickel,

g 0 oder 1 bedeuten und $L^1$, E und $Q^{12}$ jeweils die obengenannte Bedeutung besitzen.

Die Erfindung betrifft weiterhin neue Benzylverbindungen der Formel III

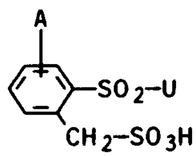 (III),

in der

A Nitro oder Amino und

$U^1$ Vinyl, Propenyl oder den Rest der Formel

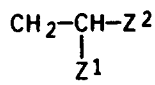 ,

worin $Z^1$ für Wasserstoff oder Methyl und $Z^2$ für eine unter alkalischen Reaktionsbedingungen abspaltbare Gruppe stehen, bedeuten,

wobei der Rest A in meta- oder para-Stellung zum Rest $CH_2$-$SO_3H$ an den Benzolring geknüpft ist.

Beispielshafte Reste U sind bereits oben genannt.

Die neuen Benzylverbindungen, die wertvolle Zwischenprodukte für die Synthese der neuen Doppelankerreaktivfarbstoffe darstellen, können nach an sich bekannten Methoden erhalten werden, wie sie beispielsweise in der EP-A-307 817 beschrieben sind.

Beispielsweise kann man eine Verbindung der Formel XXIV

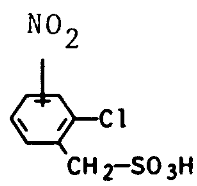 (XXIV),

mit einem Thioalkanol, z.B. 2-Thioethanol oder 1-Methyl-2-thioethanol, umsetzen.

Durch Reduktion der Nitro- zur Aminogruppe und Oxidation des Schwefelatoms zur Sulfongruppe, wobei diese Schritte in beliebiger Reihenfolge durchgeführt werden können, und anschließender Veresterung, z.B. mit Chlorsulfonsäure, gelangt man zum Benzylsulfon der Formel XXV

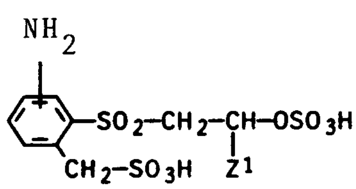 (XXV),

in der $Z^1$ die obengenannte Bedeutung besitzt.

Verbindungen der Formel XXIV können beispielsweise erhalten werden, wenn man ein Benzylchlorid der Formel XXVI

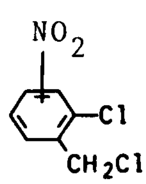 (XXVI)

mit Alkalisulfit umsetzt.

Anstelle des Schwefelsäureesters können auf an sich bekanntem Wege auch die anderen unter alkalischen Reaktionsbedingungen abspaltbaren Reste eingeführt werden.

Die Herstellung der Reaktivfarbstoffe der Formel I erfolgt z.B. dadurch, daß man einen geeigneten organischen Farbstoff oder ein geeignetes Farbstoffvorprodukt und die faseraktive Verbindung der Formel XXVII

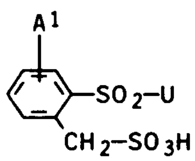

(XXVII),

in der

U die obengenannte Bedeutung besitzt und

$A^1$ den Rest

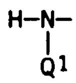

oder

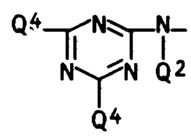

bedeuten,

worin $Q^1$, $Q^2$ und $Q^4$ jeweils die obengenannte Bedeutung besitzen, umsetzt, und daß man, im Falle der Verwendung von Farbstoffvorprodukten, die erhaltenen Zwischenverbindungen nach an sich bekannten Methoden in die gewünschten Farbstoffe umwandelt.

Wenn die Verbindung X-H eine Kupplungskomponente darstellt, gelangt man z.B. zu den erfindungsgemäßen Farbstoffen, wenn man den faserreaktiven Anker der Formel XXVIII

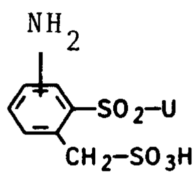

(XXVIII),

in der U die obengenannte Bedeutung besitzt, nach an sich bekannter Weise diazotiert und mit der Kupplungskomponente X-H kuppelt.

Die neuen Reaktivfarbstoffe der Formel I eignen sich in vorteilhafter Weise zum Färben oder Bedrucken von Hydroxylgruppen oder Stickstoff enthaltenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Wolle oder insbesondere von Baumwolle.

Als Färbeverfahren eignen sich die bekannten Reaktivfärbeverfahren, insbesondere Ausziehverfahren bei 40 bis 80 °C und Kaltverweilverfahren. Die neuen Farbstoffe zeichnen sich durch hohe Ausgiebigkeit und hohe Naßechtheit aus.

Die folgenden Beispiele, in denen sich Angaben über Prozente sofern nicht anders vermerkt, auf das Gewicht beziehen, sollen die Erfindung näher erläutern.

In den Tabellenbeispielen besitzen die Abkürzungen E-1 bis E-4 die folgende Bedeutung:

E-1: 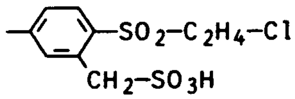

E-2: 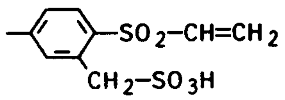

E-3: 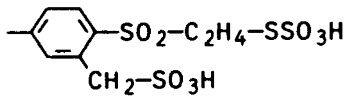

E-4: 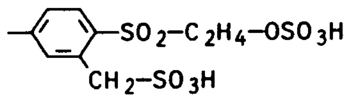

Beispiel 1

Eine Lösung von 31,4 g 4-(2'-Chlorethylsulfonyl)-3-hydroxysulfonylmethylanilin in 500 ml Wasser wurde mit 60 ml 5 N-Salzsäure und 30 ml 3,33 N $NaNO_2$-Lösung bei 0 bis 5 °C diazotiert und mit einer neutralen wäßrigen Lösung von 30,2 g 1-Hydroxynaphthalin-3,6-disulfonsäure versetzt. Durch Einstreuen von Natriumbicarbonat wurde ein pH-Wert von 5 bis 6 eingestellt. Nach beendeter Kupplung wurde der Farbstoff mit Kaliumchlorid ausgesalzen und schonend unter vermindertem Druck getrocknet. Er färbt Baumwolle in lichtechtem orangefarbenen Ton und entspricht der Formel

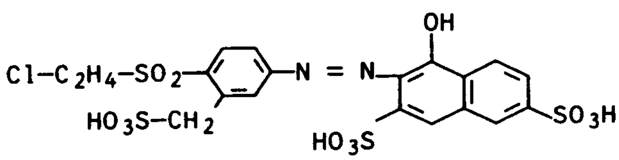

Weitere erfindungsgemäße Farbstoffe, die auf analoge Weise erhalten werden, sind in Tabelle 1 aufgeführt:

Tabelle 1

E-N = N-K

| Bsp.-Nr. | E | K | Farbton auf Baumwolle |
|---|---|---|---|
| 2 | E-1 | 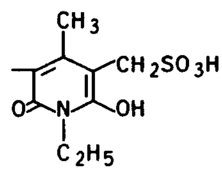 | grünstichig gelb |
| 3 | E-4 | 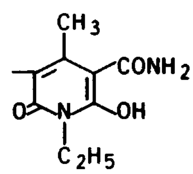 | grünstichig gelb |
| 4 | E-3 | 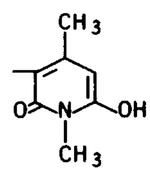 | grünstichig gelb |
| 5 | E-2 | 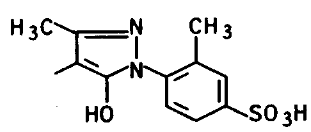 | gelb |
| 6 | E-3 | 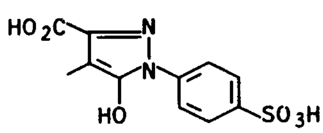 | gelb |
| 7 | E-1 | 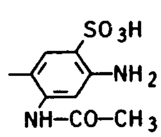 | goldgelb |

Fortsetzung - Tabelle 1

E–N = N–K

| Bsp.-Nr. | E | K | Farbton auf Baumwolle |
|---|---|---|---|
| 8 | E-1 | 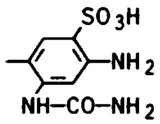 | goldgelb |
| 9 | E-3 | 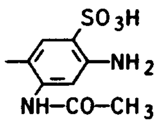 | goldgelb |
| 10 | E-2 | 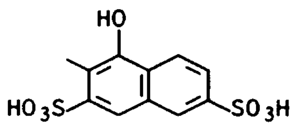 | orange |
| 11 | E-1 | 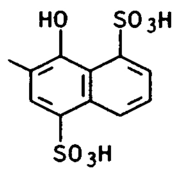 | orange |
| 12 | E-2 | 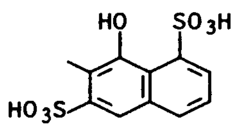 | orange |
| 13 | E-1 | 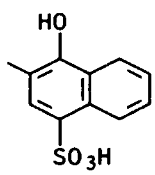 | orangerot |

Fortsetzung - Tabelle 1

E-N = N-K

| Bsp.-Nr. | E | K | Farbton auf Baumwolle |
|---|---|---|---|
| 14 | E-3 | 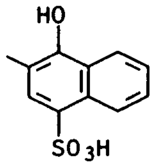 | orangerot |
| 15 | E-1 | 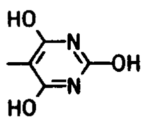 | grünstichig gelb |
| 16 | E-1 | 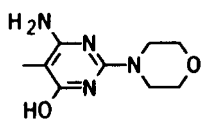 | gelb |
| 17 | E-1 | 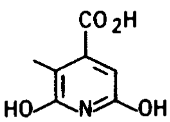 | grünstichig gelb |
| 18 | E-1 | 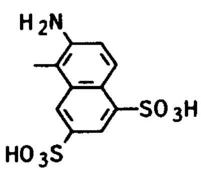 | orangerot |

Beispiel 19

31,4 g 4-(2'-Chlorethylsulfonyl)-3-hydroxysulfonylmethylanilin in 500 ml Wasser wurden bei 0 bis 5 °C salzsauer diazotiert und mit einer neutralen wäßrigen Lösung von 42,4 g 1-Benzoylamino-8-hydroxynaphthalin-4,6-disulfonsäure versetzt. Die Kupplung wurde bei einem pH-Wert von 6 bis 6,5 durch Einstreuen von Natriumbicarbonat zu Ende geführt, der gebildete Farbstoff mit Natriumchlorid ausgesalzen und schonend unter vermindertem Druck getrocknet. Er färbt Baumwolle in brillantem roten Ton mit guten Echtheiten und entspricht der Formel

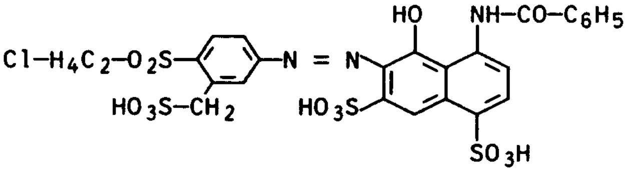

Weitere Farbstoffe, die auf analoge Weise erhalten werden, sind in Tabelle 2 aufgeführt.

Tabelle 2

E–N = N–K

| Bsp.-Nr. | E | K | Farbton auf Baumwolle |
|---|---|---|---|
| 20 | E-1 | 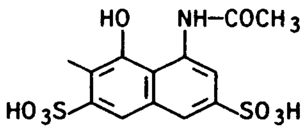 | rot |
| 21 | E-3 | 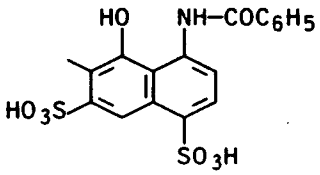 | rot |
| 22 | E-1 | 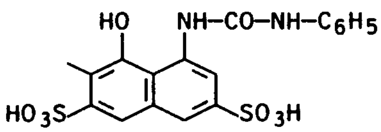 | blaustichig rot |
| 23 | E-1 | 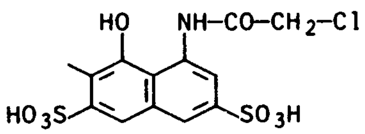 | rot |

Fortsetzung - Tabelle 2

E-N = N-K

| Bsp.-Nr. | E | K | Farbton auf Baumwolle |
|---|---|---|---|
| 24 | E-1 | 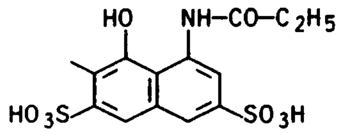 | rot |
| 25 | E-1 | 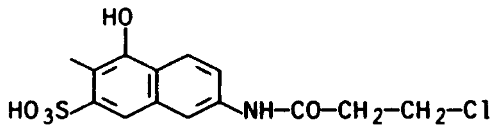 | orange |
| 26 | E-2 | 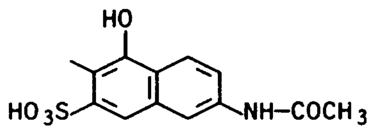 | orange |
| 27 | E-1 | 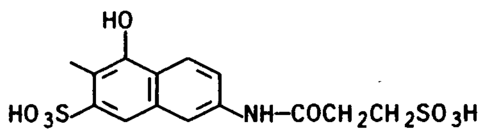 | orange |

Beispiel 28

15,7 g 4-(2'-Chlorethylsulfonyl)-3-hydroxysulfonylmethylanilin wurden in 250 ml Wasser gelöst und bei 0 °C salzsauer diazotiert. Diese Lösung tropfte man in eine Suspension von 16 g 1-Amino-8-hydroxynaphthalin-3,6-disulfonsäure in 100 ml Wasser bei einem pH-Wert von 1. Man ließ über Nacht rühren, filtrierte und tropfte 15,7 g 4-(2'-Chlorethylsulfonyl)-3-hydroxysulfonylmethylanilin - wie vorher beschrieben diazotiert - hinzu und kuppelte mit Natriumbicarbonat bei einem pH-Wert von 5,5 bis 6. Man ließ über Nacht rühren und salzte mit KCl aus. Nach schonender Trocknung erhielt man einen Farbstoff der Formel

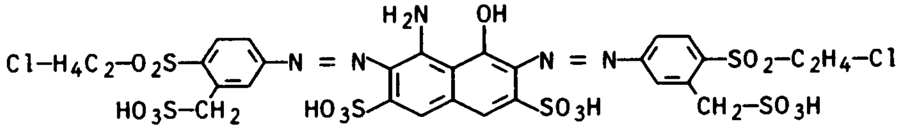

Er färbt Baumwolle in marineblauem Ton mit guten Echtheiten.

Beispiel 29

8,7 g Sulfanilsäure wurden in 200 ml $H_2O$ bei 0 °C salzsaurer diazotiert. Hierzu tropfte man eine Suspension von 13,7 g 1-Amino-8-hydroxynaphthalin-3,6-disulfonsäure (Na-Salz) in 100 ml Wasser. Der pH-Wert lag bei 1,5. Es wurde über Nacht bei Raumtemperatur gerührt.

Man tropfte dann bei 10 °C 15,7 g salzsauer diazotiertes 4-(2'-Chlorethylsulfonyl)-3-hydroxymethylanilin hinzu.

Den pH-Wert hielt man 2 Stunden mit Natriumbicarbonat auf 5,5 bis 6 und salzte mit Kaliumchlorid aus. Man saugte ab, trocknete und erhielt ein schwarzes Pulver, das Baumwolle in marineblauem Ton mit guter Lichtechtheit färbt. Der Farbstoff entspricht der Formel

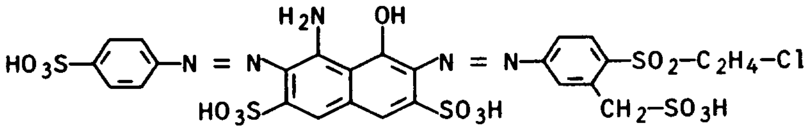

Weitere erfindungsgemäße Farbstoffe, die auf analoge Weise erhalten werden, sind in Tabelle 3 aufgeführt.

Tabelle 3

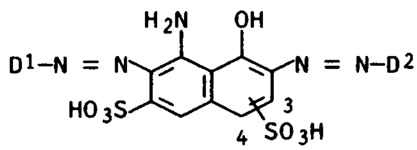

| Bsp.-Nr. | $D^1$ | $D^2$ | Stellung 3,4 | Farbton auf Baumwolle |
|---|---|---|---|---|
| 30 | 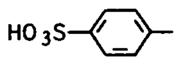 | E-1 | 4 | marineblau |
| 31 | 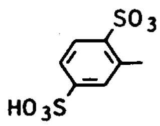 | E-2 | 3 | marineblau |
| 32 | 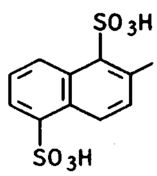 | E-1 | 3 | marineblau |
| 33 | E-2 | E-2 | 3 | marineblau |
| 34 | E-3 | E-3 | 3 | marineblau |
| 35 | E-1 | 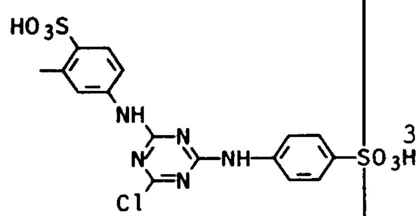 | 3 | marineblau |
| 36 | E-1 | 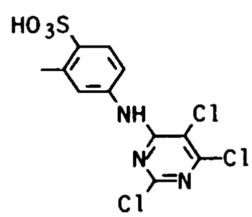 | 3 | marineblau |

Fortsetzung - Tabelle 3

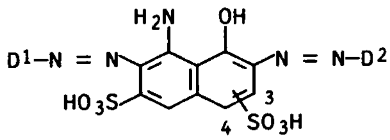

| Bsp.-Nr. | $D^1$ | $D^2$ | Stellung 3,4 | Farbton auf Baumwolle |
|---|---|---|---|---|
| 37 | E-1 | 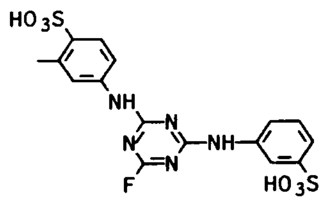 | 3 | marineblau |
| 38 | E-1 | 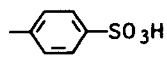 | 3 | marineblau |
| 39 | E-1 | 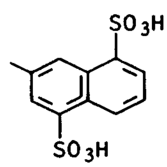 | 4 | marineblau |
| 40 | E-1 | 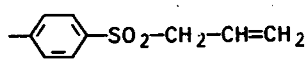 | 3 | marineblau |
| 41 | E-1 | 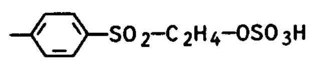 | 3 | marineblau |
| 42 | E-1 | 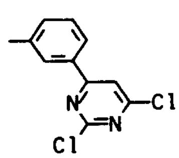 | 3 | marineblau |
| 43 | E-2 | 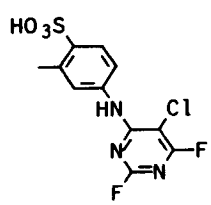 | 3 | marineblau |

Fortsetzung - Tabelle 3

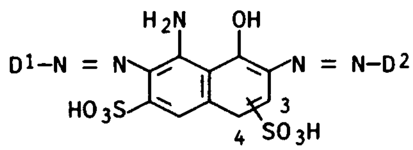

| Bsp.-Nr. | $D^1$ | $D^2$ | Stellung 3,4 | Farbton auf Baumwolle |
|---|---|---|---|---|
| 44 | E-1 | 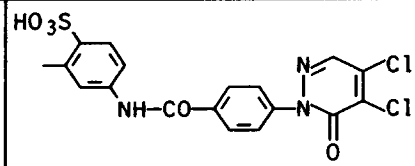 | 3 | marineblau |
| 45 | 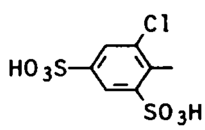 | E-1 | 3 | rotstichig marineblau |
| 46 | 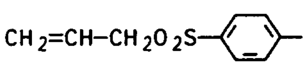 | E-1 | 3 | marineblau |
| 47 | 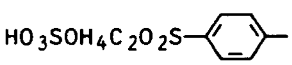 | E-1 | 3 | marineblau |
| 48 | 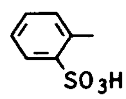 | E-1 | 3 | marineblau |
| 49 | 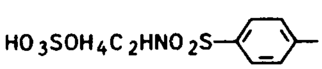 | E-1 | 3 | marineblau |
| 50 | E-1 | 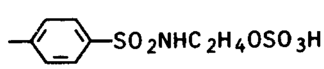 | 3 | marineblau |

Beispiel 51

Zu 25,4 g des in 800 ml Wasser neutral gelösten sekundären Kondensationsproduktes aus Anilin-3-sulfonsäure, 2,4,6-Trifluor-1,3,5-triazin und 2-Amino-5-hydroxynaphthalin-7-sulfonsäure wurden bei 0 bis 5 °C 15,7 g salzsauer diazotiertes 4-(2'-Chlorethylsulfonyl)-3-hydroxysulfonylmethylanilin in 300 ml Wasser gegeben. Durch Zusatz von Natriumbicarbonat wurde ein pH-Wert von 5 bis 6 eingestellt und der erhaltene Farbstoff mit Natriumchlorid ausgesalzen. Nach schonendem Trocknen erhielt man ein orangerotes Pulver, das Baumwolle in brillantem orangefarbenen Ton färbt. Der Farbstoff entspricht der Formel

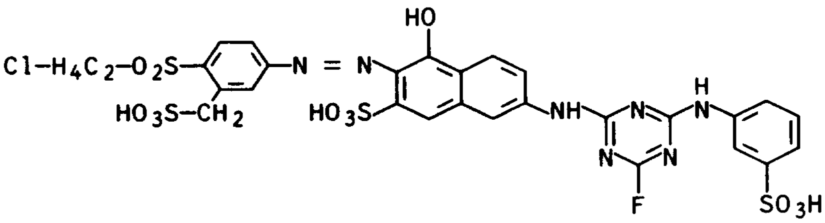

Beispiel 52

In eine neutrale Lösung von 55,2 g des sekundären Kondensationsproduktes aus 1-Amino-8-hydroxynaphthalin-3,6-disulfonsäure, Cyanurchlorid und N-Ethylanilin in 700 ml Wasser gab man 31,4 g salzsauer diazotiertes 4-(2'-Chlorethylsulfonyl)-3-hydroxysulfonylmethylanilin in 500 ml Wasser. Durch Zugabe von Natriumbicarbonat wurde die Kupplung bei einem pH-Wert von 5 bis 6 zu Ende geführt. Der gebildete Farbstoff wurde mit Natriumchlorid ausgesalzen und schonend getrocknet. Er entspricht der Formel

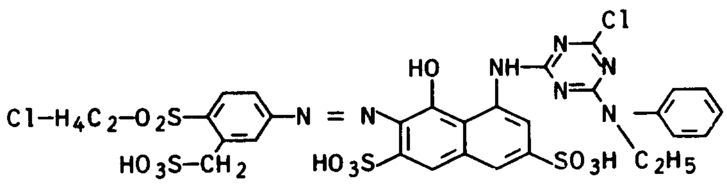

und färbt Baumwolle in brillantem roten Ton mit guten Echtheiten.

Ähnlich wie in den Beispielen 71 und 72 werden die in Tabelle 4 aufgeführten Farbstoffe erhalten.

Tabelle 4

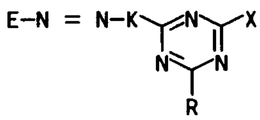

| Bsp.-Nr. | E | K | X | R | Farbton auf Baumwolle |
|---|---|---|---|---|---|
| 53 | E-1 | 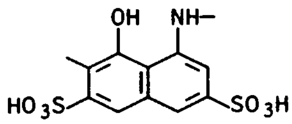 | Cl | $OCH_3$ | rot |
| 54 | E-1 | 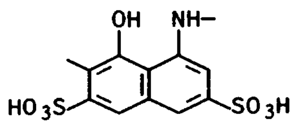 | F | 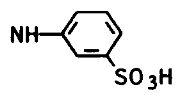 | rot |
| 55 | E-1 | 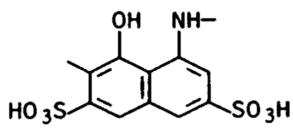 | Cl | $OCH_2CH_2OCH_3$ | rot |
| 56 | E-1 | 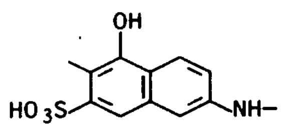 | Cl | 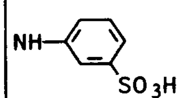 | orange |
| 57 | E-1 | 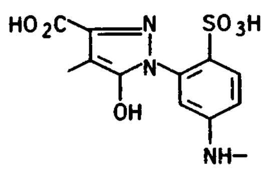 | Cl | 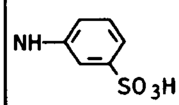 | gelb |
| 58 | E-2 | 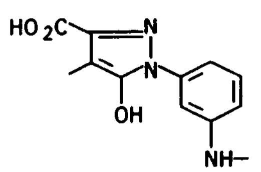 | Cl | 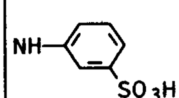 | gelb |
| 59 | E-1 | 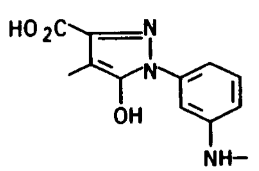 | F | 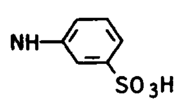 | gelb |

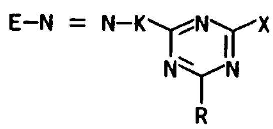

| Bsp.-Nr. | E | K | X | R | Farbton auf Baumwolle |
|---|---|---|---|---|---|
| 60 | E-1 | 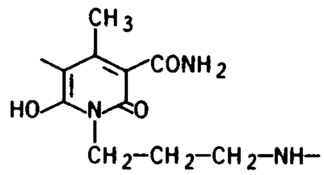 | Cl | 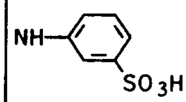 | grünstichig gelb |
| 61 | E-1 | 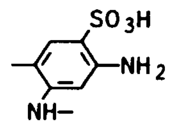 | Cl | 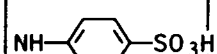 | gold-gelb |
| 62 | E-1 | 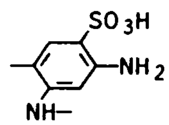 | F | 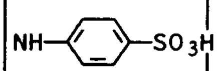 | gold-gelb |
| 63 | E-2 | 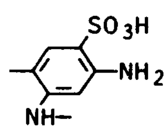 | Cl | 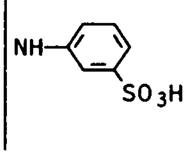 | gold-gelb |

Beispiel 64

Eine neutrale wäßrige Lösung von 31,4 g 4-(2'-Chlorethylsulfonyl)-3-hydroxysulfonylmethylanilin in 500 ml Wasser wurde mit einer Suspension von 19,3 g Cyanurchlorid in 250 ml Eiswasser versetzt und 2,5 Stunden bei 0 bis 5 °C gerührt, wobei durch Zugabe von Natriumbicarbonat ein pH-Wert von 5 bis 6 eingehalten wurde. Man filtrierte, gab die Suspension zu einer bei 40 °C und pH 5 bis 6 gerührten Lösung von 16,9 g 1,3-Diaminobenzol-4-sulfonsäure in 100 ml Wasser und hielt 2 Stunden bei 35 bis 40 °C und pH 3 bis 4.

Nach beendeter Umsetzung wurde bei 0 bis 5 °C durch Zugabe von 30 ml 3,33 wäßriger Natriumnitritlösung und 60 ml 5 N Salzsäure diazotiert und auf 27,6 g 1,4-Dimethyl-3-hydroxysulfonylmethyl-6-hydroxypyridon-2 gekuppelt. Der erhaltene Farbstoff wurde mit Natriumchlorid ausgesalzen und schonend unter vermindertem Druck getrocknet. Er färbt Baumwolle in brillantem, echten, grünstichig gelben Ton und entspricht der Formel

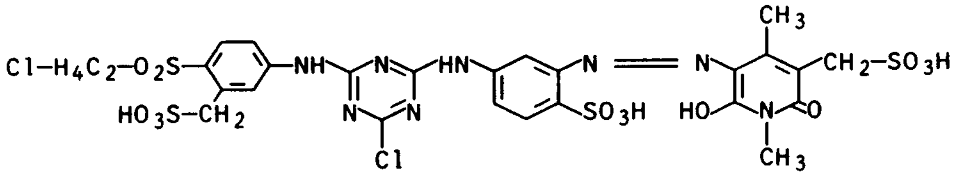

Beispiel 65

33 g des Natriumsalzes des Farbstoffs der Formel

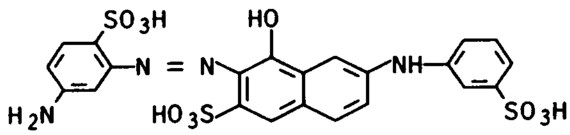

wurden bei pH 6 und 40 °C in Wasser gelöst und mit 31,6 g des in Beispiel 81 beschriebenen Kondensationsproduktes aus Cyanurchlorid und 4-(2'-Chlorethylsulfonyl)-3-hydroxysulfonylmethylanilin gelöst in 1000 ml Wasser, versetzt und weitere 2 Stunden bei 40 °C gerührt, wobei durch Einstreuen von Natriumbicarbonat ein pH-Wert von 5 bis 6 eingehalten wird. Der mit Kaliumchlorid ausgefällte und schonend getrocknete Farbstoff ergibt auf Baumwolle eine braune Färbung und entspricht der Formel

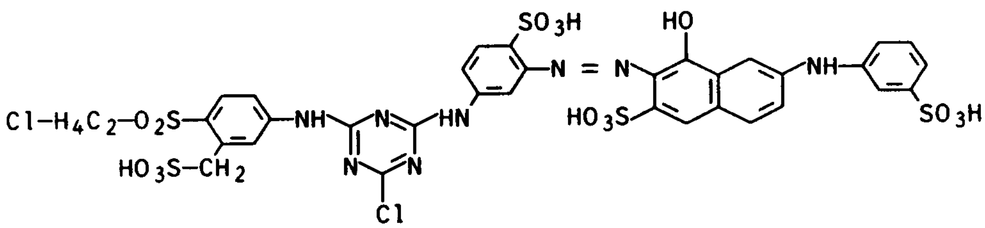

In ähnlicher Weise werden die in Tabelle 5 beschriebenen Farbstoffe erhalten.

Tabelle 5

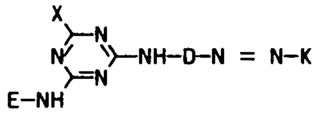

| Bsp.-Nr. | E | X | D | K | Farbton auf Baumwolle |
|---|---|---|---|---|---|
| 66 | E-1 | Cl | 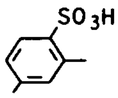 | 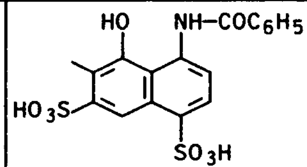 | rot |
| 67 | E-1 | F | 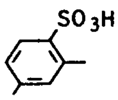 | 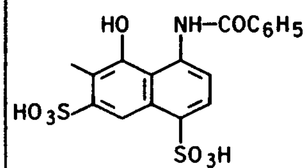 | rot |
| 68 | E-1 | Cl | 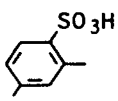 | 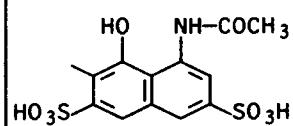 | rot |
| 69 | E-1 | Cl | 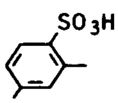 | 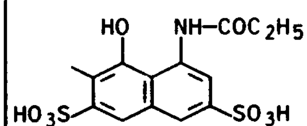 | rot |
| 70 | E-1 | Cl | 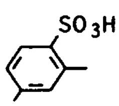 | 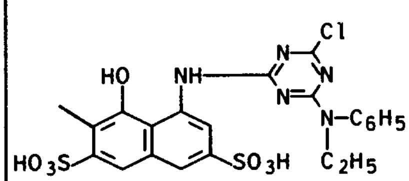 | rot |
| 71 | E-1 | Cl | 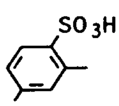 | 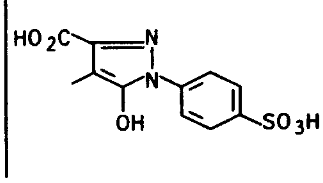 | gelb |

Fortsetzung - Tabelle 5

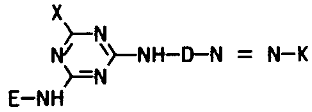

| Bsp.-Nr. | E | X | D | K | Farbton auf Baumwolle |
|---|---|---|---|---|---|
| 72 | E-1 | Cl | 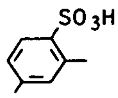 | 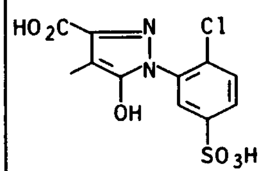 | gelb |
| 73 | E-1 | Cl | 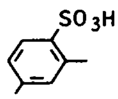 | 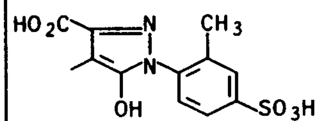 | gelb |
| 74 | E-1 | Cl | 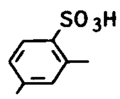 | 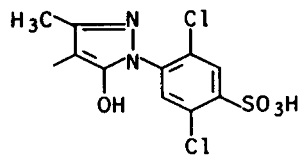 | gelb |
| 75 | E-1 | Cl | 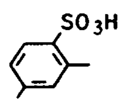 | 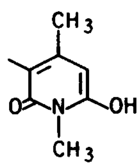 | grünstichig gelb |
| 76 | E-1 | Cl | 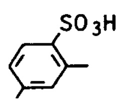 | 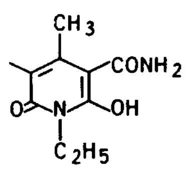 | grünstichig gelb |
| 77 | E-1 | Cl | 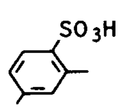 | 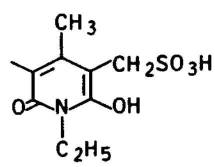 | grünstichig gelb |

Fortsetzung - Tabelle 5

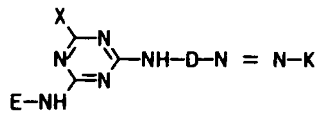

| Bsp.-Nr. | E | X | D | K | Farbton auf Baumwolle |
|---|---|---|---|---|---|
| 78 | E-1 | Cl | 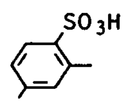 | 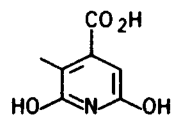 | grünstichig gelb |
| 79 | E-1 | Cl | 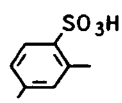 | 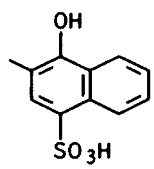 | orangerot |
| 80 | E-1 | Cl | 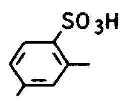 | 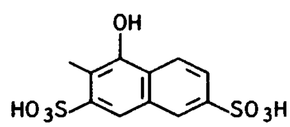 | orange |
| 81 | E-1 | Cl | 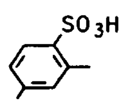 | 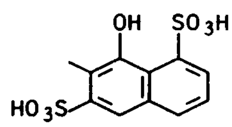 | orange |
| 82 | E-1 | Cl | 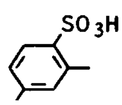 | 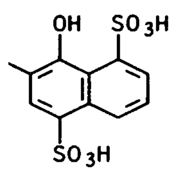 | orangerot |
| 83 | E-1 | Cl | 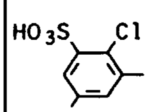 | 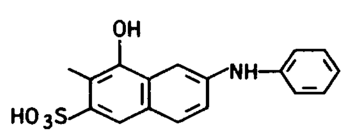 | braun |
| 84 | E-1 | Cl | 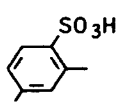 | 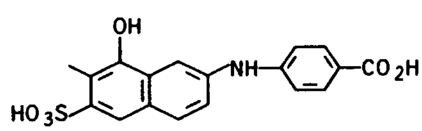 | braun |

Fortsetzung - Tabelle 5

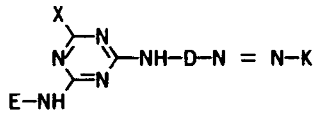

| Bsp.-Nr. | E | X | D | K | Farbton auf Baumwolle |
|---|---|---|---|---|---|
| 85 | E-1 | Cl | 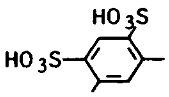 | 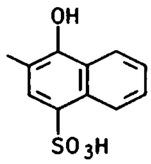 | orangerot |
| 86 | E-1 | Cl | 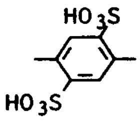 | 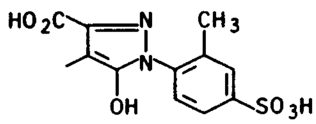 | goldgelb |
| 87 | E-1 | Cl | 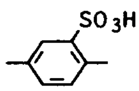 | 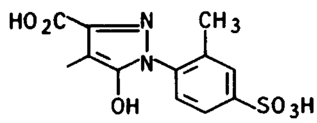 | goldgelb |
| 88 | E-1 | Cl | 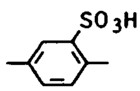 | 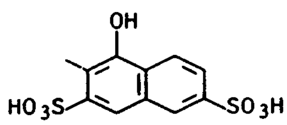 | rot |
| 89 | E-1 | Cl | 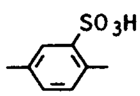 | 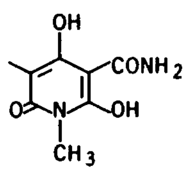 | goldgelb |
| 90 | E-1 | Cl | 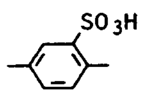 | 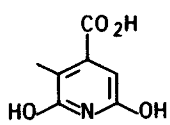 | goldgelb |
| 91 | E-1 | Cl | 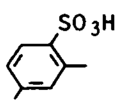 | 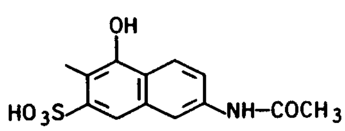 | orange |

Fortsetzung - Tabelle 5

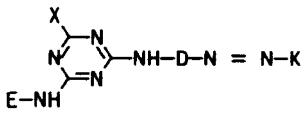

| Bsp.-Nr. | E | X | D | K | Farbton auf Baumwolle |
|---|---|---|---|---|---|
| 92 | E-1 | F | 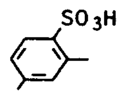 | 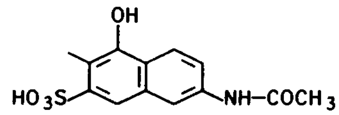 | orange |
| 93 | E-1 | Cl | 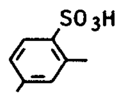 | 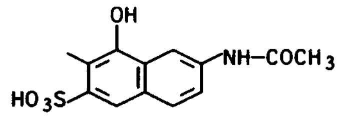 | orange |
| 94 | E-1 | Cl | 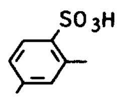 | 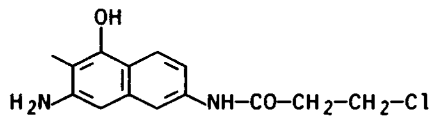 | orange |
| 95 | E-1 | Cl | 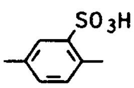 | 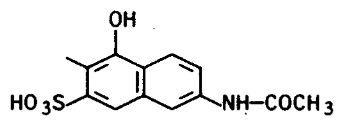 | rot |
| 96 | E-2 | Cl | 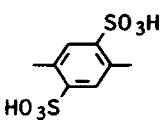 | 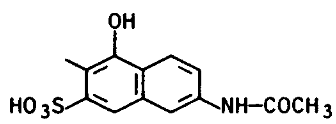 | rot |
| 97 | E-1 | Cl | 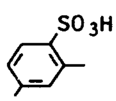 | 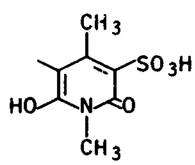 | grünstichig gelb |
| 98 | E-1 | Cl | 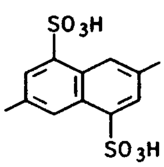 | 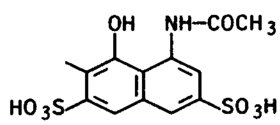 | blaustichig rot |

Fortsetzung - Tabelle 5

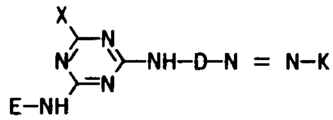

| Bsp.-Nr. | E | X | D | K | Farbton auf Baumwolle |
|---|---|---|---|---|---|
| 99 | E-1 | Cl | 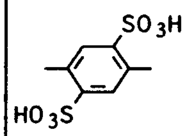 | 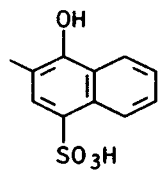 | rot |
| 100 | E-1 | Cl | 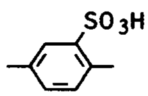 | 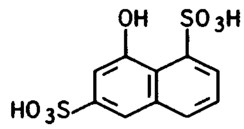 | rot |
| 101 | E-1 | Cl | 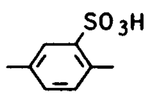 | 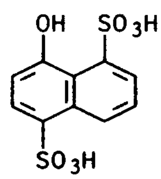 | rot |

Beispiel 102

27,7 g des durch Kupplung von diazotierter 2-Aminonaphthalin-3,6,8-trisulfonsäure mit 3-Aminophenylharnstoff erhaltenen Aminoazofarbstoffs wurden in 250 ml Wasser neutral gelöst und bei 40 °C mit einer (wie in Bsp. 81 hergestellten) neutralen wäßrigen Lösung von 21,4 g des Kondensationsproduktes aus Cyanurchlorid und 4-(2'-Chlorethylsulfonyl)-3-hydroxy-sulfonylmethylanilin in 1000 ml Wasser versetzt und 2 Stunden bei 40 °C gerührt. Dabei wurde durch Einstreuen von Natriumbicarbonat ein pH-Wert von 5 bis 6 eingehalten. Sobald dünnschichtchromatographisch keine freien Aminogruppen mehr nachweisbar waren, wurde mit Kaliumchlorid ausgesalzen und schonend unter vermindertem Druck getrocknet. Das Produkt entspricht der Formel

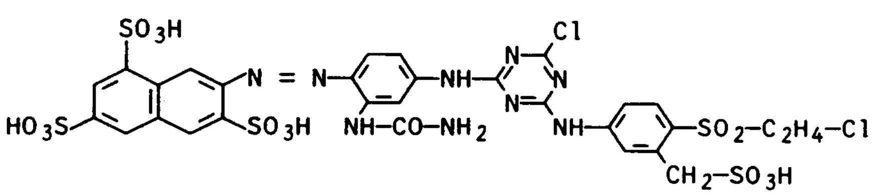

und färbt Baumwolle in echtem, goldgelben Ton mit guten Echtheiten.

Beispiel 103

Die neutrale wäßrige Lösung von 21,4 g des in Bsp. 64 beschriebenen primären Kondensationsproduktes aus Cyanurchlorid und 4-(2'-Chlorethylsulfonyl)-3-hydroxysulfonylmethylanilin wurde mit 15 g 1-Amino-8-hydroxynaphthalin-3,6-disulfonsäure versetzt und 2 Stunden bei 30 °C gerührt, wobei durch Zugabe von

Natriumbicarbonat ein pH-Wert von 5 bis 6 eingehalten wurde. Man kühlte mit Eis auf 0 bis 5 °C, gab 8,7 g diazotierte Anilin-2-sulfonsäure zu und brachte die Kupplung durch Zusatz von Natriumbicarbonat bei pH 5 bis 6 zu Ende. Der entstandene Farbstoff besitzt die Strukturformel

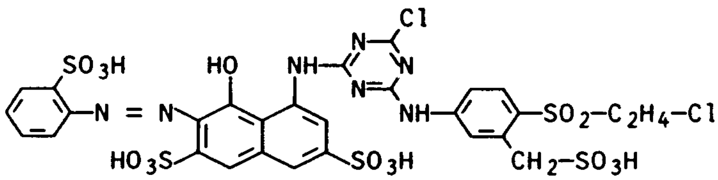

und färbt Baumwolle in echtem, brillanten roten Ton.

In analoger Weise werden die in Tabelle 6 aufgeführten Farbstoffe erhalten.

Tabelle 6

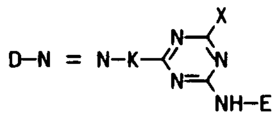

| Bsp.-Nr. | D | K | X | E | Farbton auf Baumwolle |
|---|---|---|---|---|---|
| 104 | 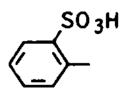 | 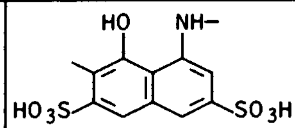 | Cl | E-1 | rot |
| 105 | 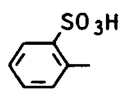 | 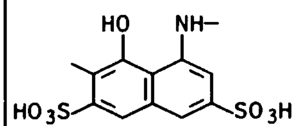 | F | E-1 | rot |
| 106 | 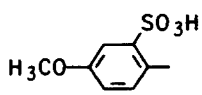 | 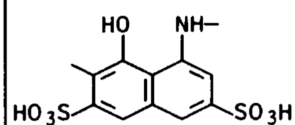 | Cl | E-1 | bordo-rot |
| 107 | 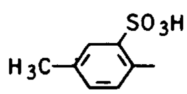 | 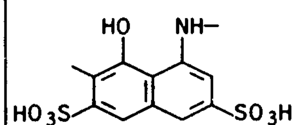 | Cl | E-1 | blaustichig rot |
| 108 | 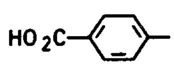 | 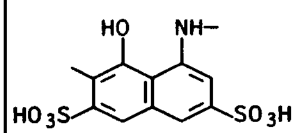 | Cl | E-1 | blaustichig rot |
| 109 | 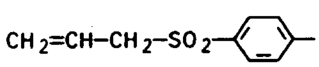 | 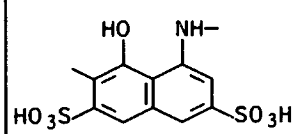 | Cl | E-1 | rot |
| 110 | 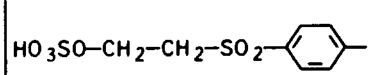 | 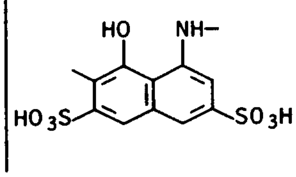 | Cl | E-1 | rot |

Fortsetzung - Tabelle 6

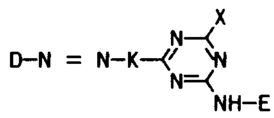

| Bsp.-Nr. | D | K | X | E | Farbton auf Baumwolle |
|---|---|---|---|---|---|
| 111 | 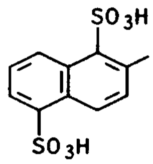 | 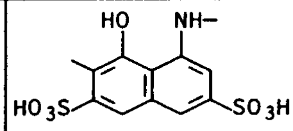 | Cl | E-1 | blaustichig rot |
| 112 | 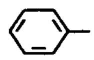 | 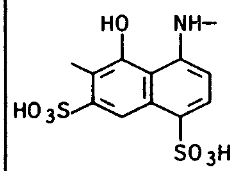 | Cl | E-1 | rot |
| 113 | 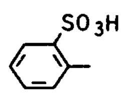 | 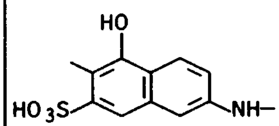 | Cl | E-1 | orange |
| 114 | 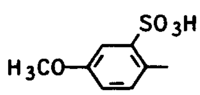 | 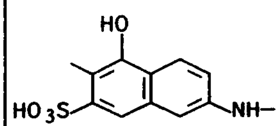 | Cl | E-1 | orange |
| 115 | 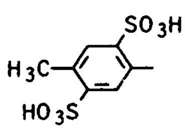 | 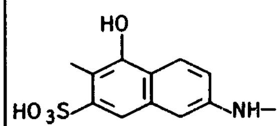 | Cl | E-1 | orange |
| 116 | 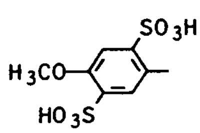 | 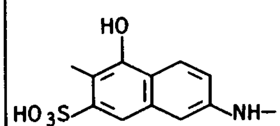 | Cl | E-1 | orange |

Fortsetzung - Tabelle 6

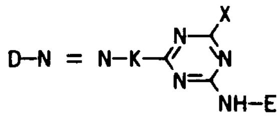

| Bsp.-Nr. | D | K | X | E | Farbton auf Baumwolle |
|---|---|---|---|---|---|
| 117 | 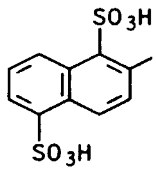 | 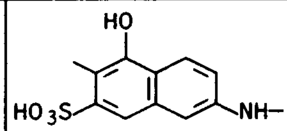 | Cl | E-1 | orange |
| 118 | 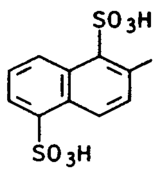 | 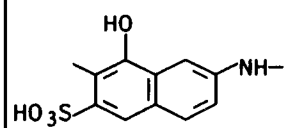 | Cl | E-1 | scharlach-rot |
| 119 | 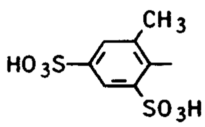 | 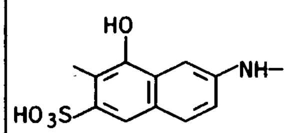 | Cl | E-1 | scharlach-rot |
| 120 | 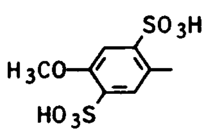 | 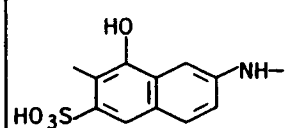 | Cl | E-1 | scharlach-rot |
| 121 | 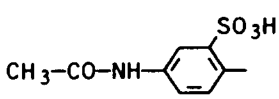 | 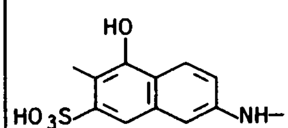 | Cl | E-1 | rot |
| 122 | 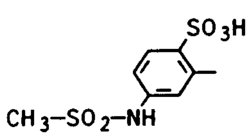 | 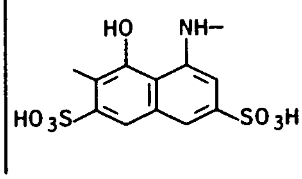 | Cl | E-1 | rot |

Fortsetzung - Tabelle 6

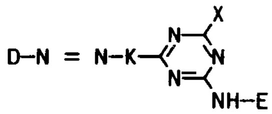

| Bsp.-Nr. | D | K | X | E | Farbton auf Baumwolle |
|---|---|---|---|---|---|
| 123 | 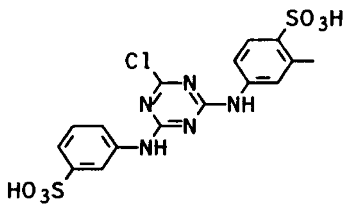 | 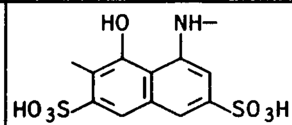 | Cl | E-1 | rot |
| 124 | 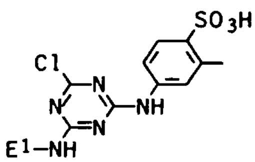 | 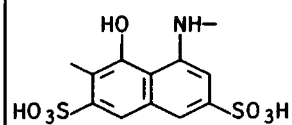 | Cl | E-1 | rot |
| 125 | 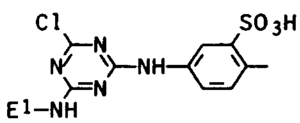 | 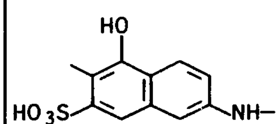 | Cl | E-1 | rot |
| 126 | 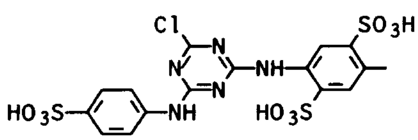 | 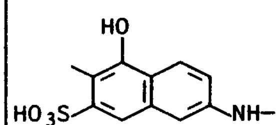 | Cl | E-1 | rot |
| 127 | 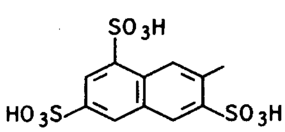 | 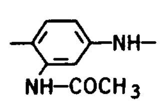 | Cl | E-1 | goldgelb |
| 128 | 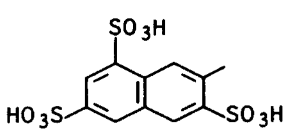 | 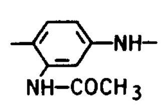 | F | E-1 | goldgelb |

Fortsetzung - Tabelle 6

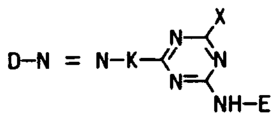

| Bsp.-Nr. | D | K | X | E | Farbton auf Baumwolle |
|---|---|---|---|---|---|
| 129 | 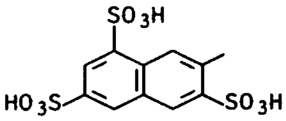 | 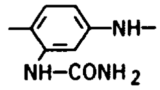 | F | E-1 | goldgelb |
| 130 | 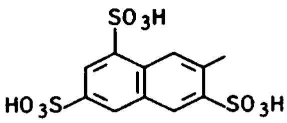 | 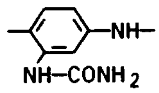 | Cl | E-2 | goldgelb |
| 131 | 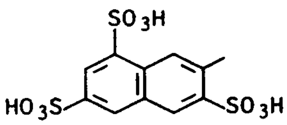 | 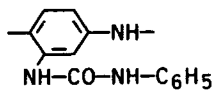 | Cl | E-1 | goldgelb |
| 132 | 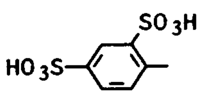 | 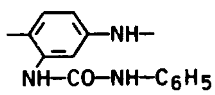 | Cl | E-1 | gelb |
| 133 | 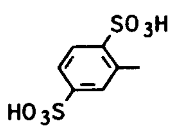 | 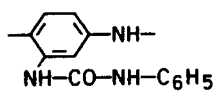 | Cl | E-1 | gelb |
| 134 | 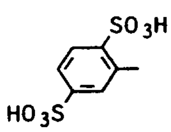 | 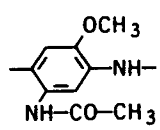 | Cl | E-1 | goldgelb |
| 135 | 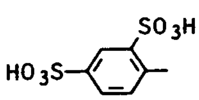 | 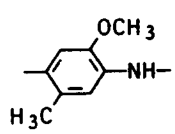 | Cl | E-1 | goldgelb |

Fortsetzung - Tabelle 6

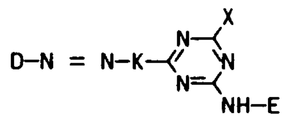

| Bsp.-Nr. | D | K | X | E | Farbton auf Baumwolle |
|---|---|---|---|---|---|
| 136 | 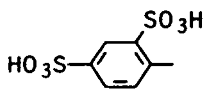 | 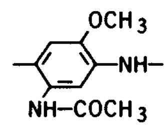 | Cl | E-1 | goldgelb |
| 137 | 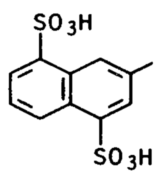 | 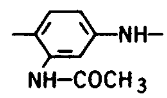 | Cl | E-1 | goldgelb |
| 138 | 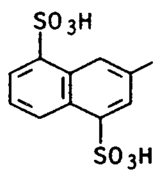 | 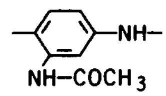 | F | E-1 | goldgelb |
| 139 | 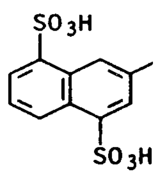 | 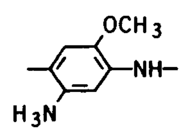 | Cl | E-1 | goldgelb |

Beispiel 140

8,7 g Sulfanilsäure wurden in 250 ml Wasser salzsauer diazotiert. Man tropfte eine Suspension von 13,7 g 1-Amino-8-hydroxy-naphthalin-3,6-disulfonsäure in 100 ml Wasser hinzu und rührte 12 Stunden bei Raumtemperatur und einem pH-Wert von 1,5. Man kühlte auf 10 °C ab und setzte eine Diazokomponente zu, die durch salzsaure Diazotierung von 56,4 g des sekundären Kondensationsprodukts aus 4-(2'-Chlorethylsulfonyl)-3-hydroxysulfonylmethylanilin, Cyanurchlorid und 1,3-Phenylendiamin-4-sulfonsäure in 750 ml Wasser erhalten wurde.

Anschließend ließ man bei einem pH-Wert von 5,5 bis 6 durch Zugabe von Natriumhydrogencarbonat über Nacht ausreagieren. Man salzte mit 200 g Natriumchlorid aus und erhielt nach dem Trocknen einen Farbstoff der Formel

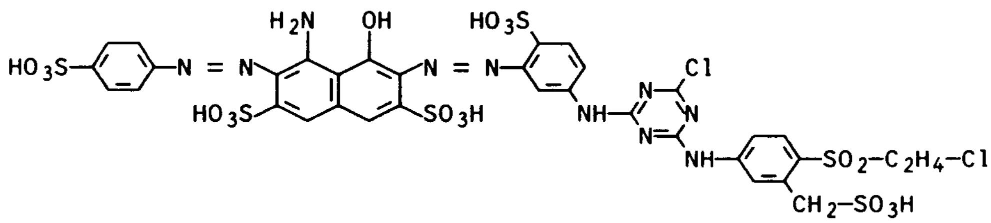

der Baumwolle in echtem, marineblauen Ton färbt.

Beispiel 141

Man ersetzte in Bsp. 140 1-Amino-8-hydroxynaphthalin-3,6-disulfonsäure durch 1-Amino-8-hydroxynaphthalin-4,6-disulfonsäure und erhielt einen Farbstoff mit ähnlichen Eigenschaften.

Tabelle 7 enthält weitere Farbstoffbeispiele, die analog Beispiel 140 erhalten werden.

Tabelle 7

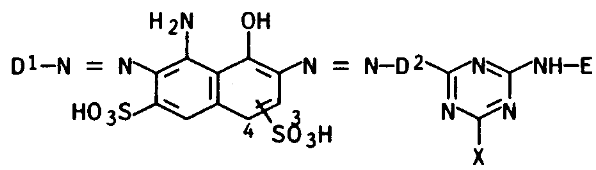

| Bsp.-Nr. | $D^1$ | Stellung 3/4 | $D^2$ | X | E | Farbton auf Baumwolle |
|---|---|---|---|---|---|---|
| 142 | 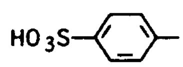 | 3 | 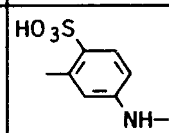 | F | E-1 | marineblau |
| 143 | 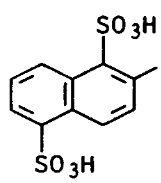 | 3 | 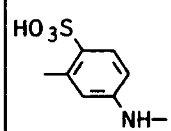 | Cl | E-1 | marineblau |
| 144 | 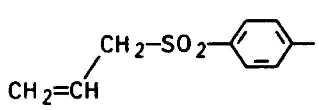 | 4 | 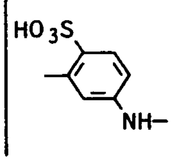 | Cl | E-1 | marineblau |

Fortsetzung - Tabelle 7

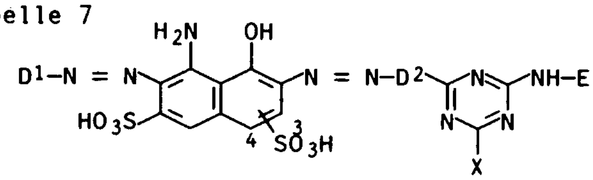

| Bsp.-Nr. | D1 | Stellung 3/4 | D2 | X | E | Farbton auf Baumwolle |
|---|---|---|---|---|---|---|
| 145 | 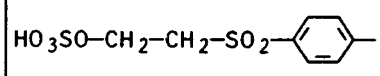 | 3 | 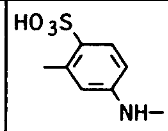 | Cl | E-1 | marineblau |
| 146 | 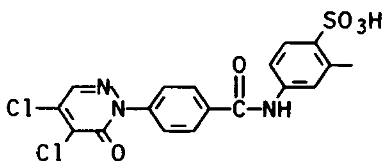 | 3 | 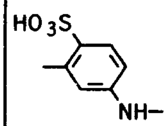 | Cl | E-1 | marineblau |
| 147 | 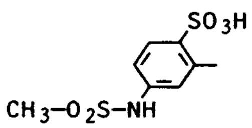 | 3 | 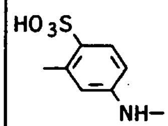 | Cl | E-1 | marineblau |
| 148 | 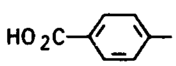 | 3 | 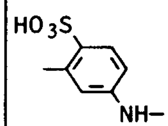 | Cl | E-1 | marineblau |
| 149 | 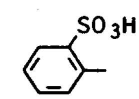 | 3 | 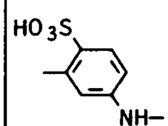 | Cl | E-1 | marineblau |
| 150 | 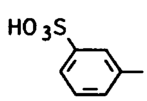 | 3 | 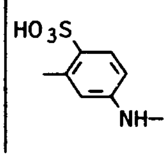 | Cl | E-1 | marineblau |

Beispiel 151

Zu der Lösung von 28,2 g des analog Beispiel 135 hergestellten und diazotierten sekundären Kondensationsproduktes von 1,3-Phenylendiamin-4-sulfonsäure, Cyanurchlorid und 4-(2'-Chlorethylsulfonyl)-3-hydroxysulfonylmethylanilin in 750 ml Wasser gab man bei 0 bis 5 °C 15,9 g 1-Amino-8-hydroxynaphthalin-3,6-disulfonsäure, suspendiert in 100 ml Wasser. Mit Natriumformiat wurde ein pH-Wert von 2,5 bis 3 gehalten. Man ließ 12 Stunden bei Raumtemperatur rühren, gab dann bei 10 °C 8,7 g salzsauer diazotierte Anilin-4-sulfonsäure, in 200 ml Wasser gelöst, hinzu und hielt den pH-Wert mit Natriumbicarbonat bei 6 bis 6,5. Nach dem Aussalzen mit Natriumchlorid erhielt man einen Farbstoff der Formel

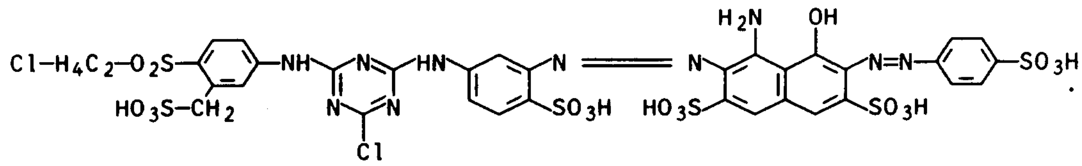

Die in Tabelle 8 aufgeführten Farbstoffe werden in analoger Weise erhalten.

Tabelle 8

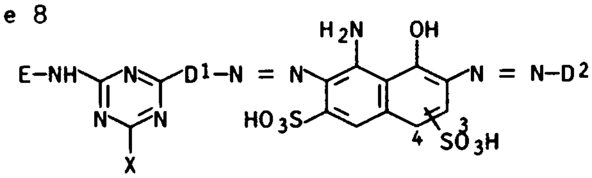

| Bsp.-Nr. | E | X | $D^1$ | Stellung 3/4 | $D^2$ | Farbton auf Baumwolle |
|---|---|---|---|---|---|---|
| 152 | E-1 | Cl | 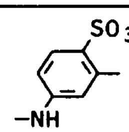 | 3 | 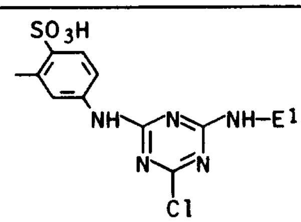 | marineblau |
| 153 | E-1 | Cl | 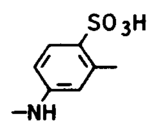 | 3 | 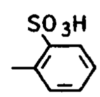 | marineblau |
| 154 | E-1 | Cl | 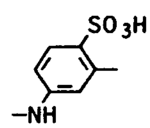 | 3 | 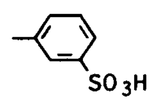 | marineblau |
| 155 | E-2 | Cl | 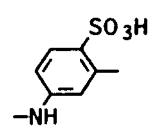 | 3 | 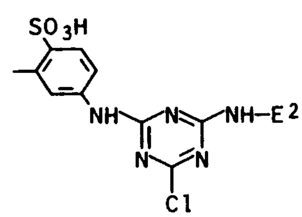 | marineblau |

Fortsetzung - Tabelle 8

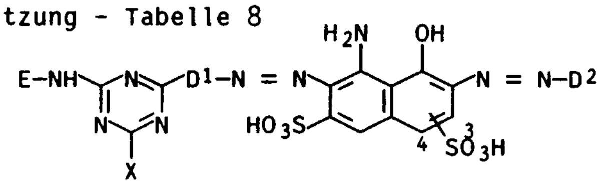

| Bsp.-Nr. | E | X | D1 | Stellung 3/4 | D2 | Farbton auf Baumwolle |
|---|---|---|---|---|---|---|
| 156 | E-1 | Cl | 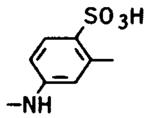 | 4 | 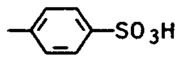 | marineblau |
| 157 | E-1 | Cl | 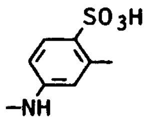 | 3 | 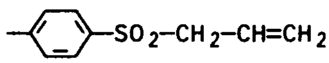 | marineblau |
| 158 | E-1 | Cl | 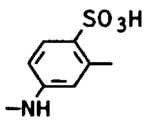 | 4 | 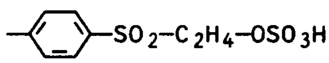 | marineblau |

Beispiel 159

38,6 g des bekannten Farbstoffs der Formel

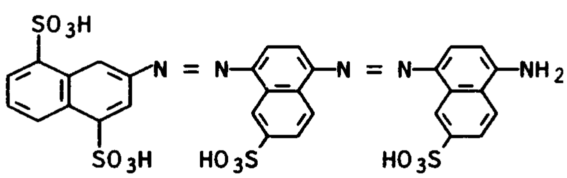

wurden bei einem pH-Wert von 7 in 400 ml Wasser bei 40 °C vorgelegt und mit 21,4 g des wie in Beispiel 64 hergestellten primären Kondensationsproduktes aus Cyanurchlorid und 4-(2'-Chlorethylsulfonyl)-3-hydroxysulfonylmethylanilinin 500 ml Wasser versetzt. Man rührte bei 40 °C und einem pH-Wert von 5 bis 6 bis dünnschichtchromatographisch keine freien Aminogruppen mehr nachweisbar waren. Der nach Aussalzen mit Kaliumchlorid erhaltene Farbstoff entspricht der Formel

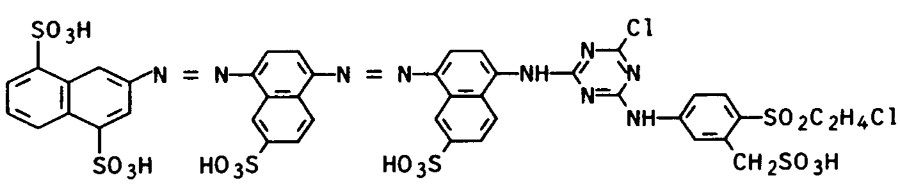

und färbt Baumwolle in rotbraunem Ton.

Tabelle 9 enthält Farbstoffe, die auf analoge Weise erhalten werden.

Tabelle 9

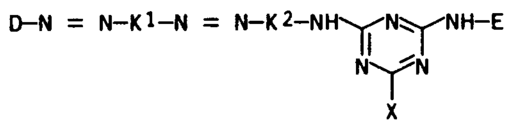

| Bsp.-Nr. | D | $K^1$ | $K^2$ | X | E | Farbton auf Baumwolle |
|---|---|---|---|---|---|---|
| 160 | 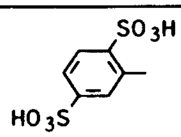 | 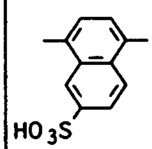 | 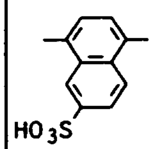 | Cl | E-1 | rotstichig braun |
| 161 | 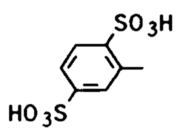 | 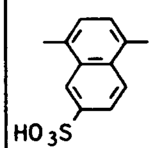 | 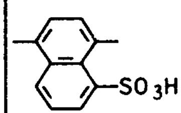 | Cl | E-1 | rotstichig braun |
| 162 | 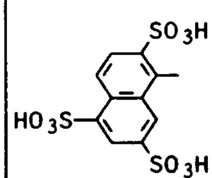 | 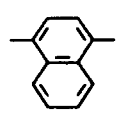 | 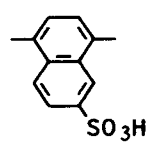 | Cl | E-1 | rotstichig braun |
| 163 | 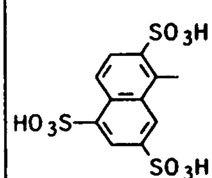 | 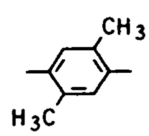 | 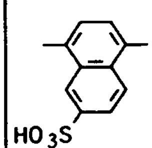 | Cl | E-1 | gelbstichig braun |
| 164 | 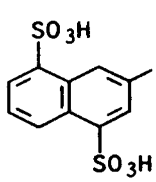 | 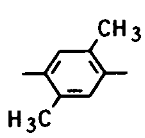 | 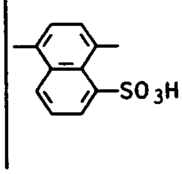 | Cl | E-2 | gelbstichig braun |

Fortsetzung - Tabelle 9

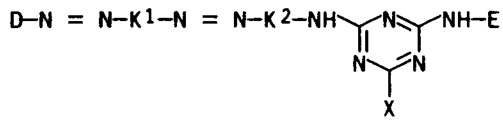

| Bsp.-Nr. | D | $K^1$ | $K^2$ | X | E | Farbton auf Baumwolle |
|---|---|---|---|---|---|---|
| 165 | 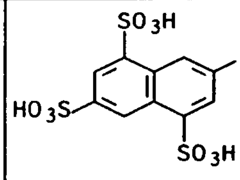 | 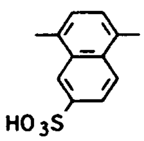 | 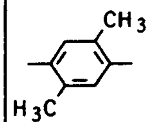 | F | E-1 | gelbstichig braun |
| 166 | 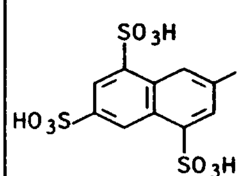 | 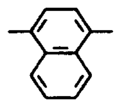 | 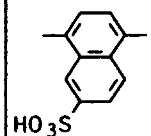 | Cl | E-1 | rotstichig braun |
| 167 | E1 | 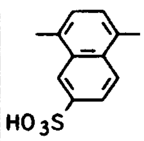 | 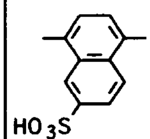 | Cl | 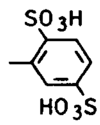 | rotbraun |
| 168 | 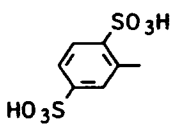 | 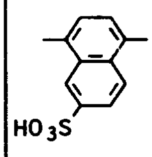 | 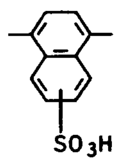 | Cl | E-1 | rotbraun |
| 169 | 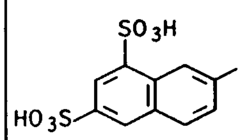 | 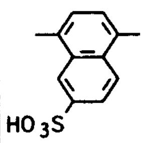 | 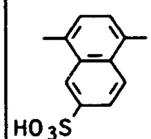 | Cl | E-1 | rotbraun |
| 170 | 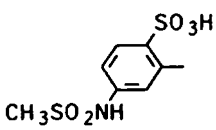 | 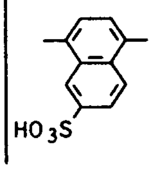 | 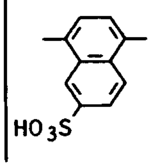 | Cl | E-1 | rotbraun |

Beispiel 171

64,8 g des bekannten Farbstoffs der Formel

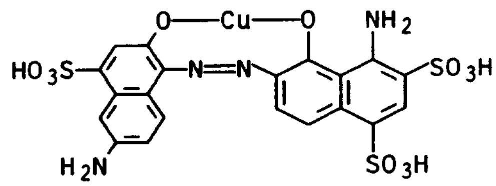

wurden in 700 ml Wasser angerührt. Bei einem pH-Wert von 5,5 bis 6 und 40 °C setzte man über 2 Stunden 21,4 g des in Beispiel 64 beschriebenen primären Kondensationsproduktes aus Cyanurchlorid und 4-(2'-Chlorethylsulfonyl)-3-hydroxysulfonylmethylanilin, gelöst in 800 ml Wasser, hinzu und hielt weitere 30 Minuten bei einem pH-Wert von 5,5 bis 6 und 40 bis 45 °C. Nach beendeter Umsetzung wurde mit Natriumchlorid ausgesalzen, abfiltriert und unter vermindertem Druck getrocknet. Der resultierende Farbstoff entspricht der Formel

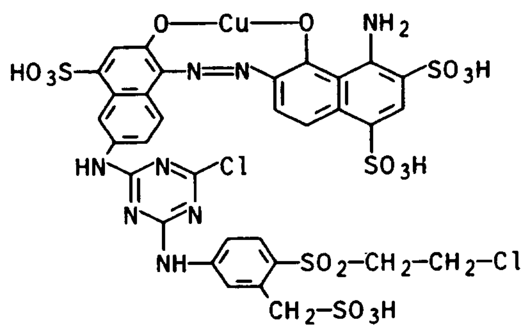

und färbt Baumwolle in echtem blauen Ton.

Beispiel 172

Zu einer Suspension von 75 g des bekannten Dichlortriazinfarbstoffs der Formel

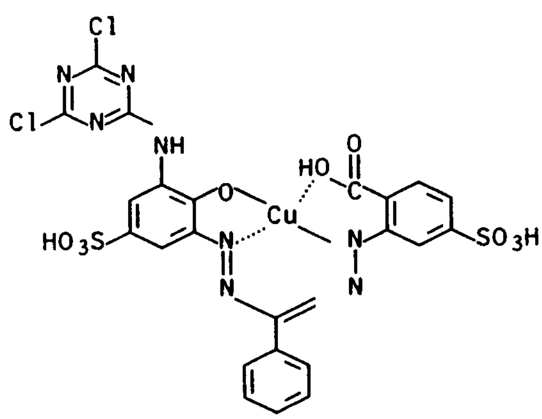

in 600 ml Wasser wurde im Neutralen eine Lösung von 49 g 4-(2'-Chlorethylsulfonyl)-3-hydroxysulfonylmethylanilin in 600 ml Wasser eingetragen. Die Suspension wurde auf 40 bis 45 °C erwärmt und der pH-Wert wurde durch Zusatz von $NaHCO_3$ am Neutralpunkt gehalten. Nach 2,5 Stunden wurde der gebildete Farbstoff, entsprechend der Formel

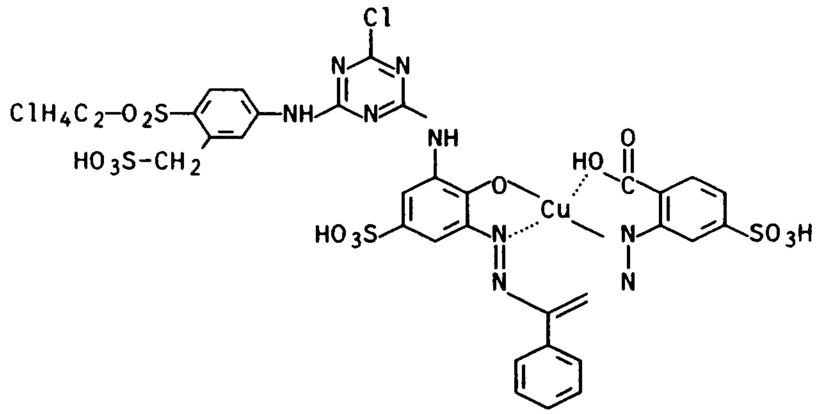

,

mit 250 g Natriumchlorid ausgesalzen, abfiltriert und getrocknet. Das erhaltene dunkelblaue Farbstoffpulver färbt Baumwolle in klarem blauen Ton. Die Färbungen sind licht- und naßecht, sie weisen eine bemerkenswerte Stabilität gegenüber oxidativen Einflüssen auf.

Beispiel 173

Analog der in Beispiel 172 beschriebenen Methode kann der Farbstoff der Formel

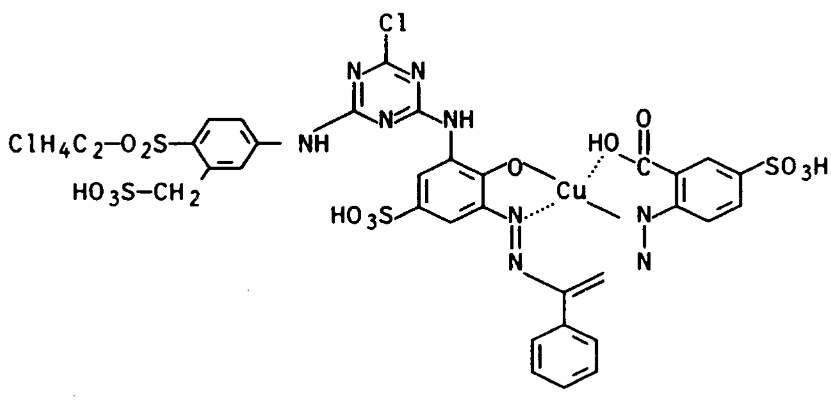

erhalten werden.

Beispiel 174

43,4 g 88 %ige 1-Amino-4-bromanthrachinon-2-sulfonsäure, 34,5 g des Amins der Formel

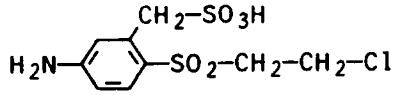

1,5 g Cu-Pulver, 0,75 g Cu-II-Sulfat und 50,4 g Natriumhydrogencarbonat wurden 120 Stunden auf 65 bis 70 °C erhitzt. Nach vollständigem Umsatz (Dünnschichtchromatographie) wurde heiß filtriert und das Filtrat mit konz. Salzsäure auf einen pH-Wert von 1 gestellt. Der ölige Rückstand wurde bei 0 bis 5 °C durch Ausrühren mit 100 ml Ethanol kristallisiert, das kristalline Produkt isoliert, mit Ethanol gewaschen und getrocknet. Man erhielt 45 g einer Verbindung der Formel

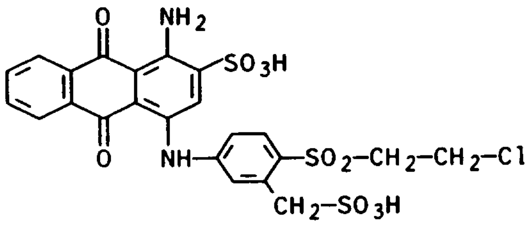

In analoger Weise werden die in Tabelle 10 aufgeführten Verbindungen der Formel

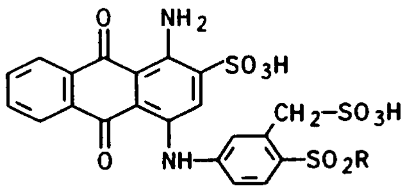

erhalten.

Tabelle 10

| Bsp.-Nr. | R |
|---|---|
| 175 | $CH=CH_2$ |
| 176 | $CH_2CH_2OH$ |
| 177 | $CH_2CH_2SSO_3H$ |

Beispiel 178

10 g der in Bsp. 174 erhaltenen Verbindung der Formel

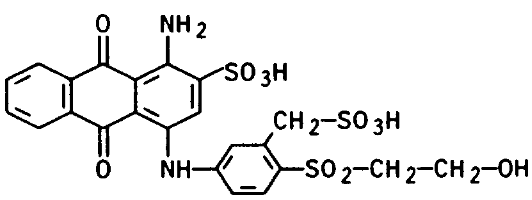

wurden in 40 g Chlorsulfonsäure 3 Stunden bei 20 bis 25 °C gerührt. Die Schmelze wurde auf 400 g Eis/Wasser gefällt und mit Natriumhydrogencarbonat auf einen pH-Wert von 5 gestellt. Nach Sprühtrocknung der Lösung wurde ein Produkt isoliert, das naben Salz den Farbstoff der Formel

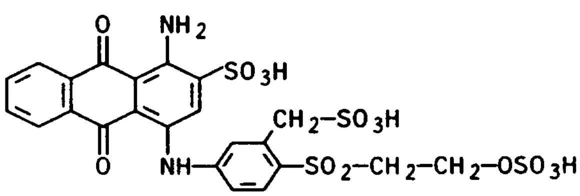

enthielt. Auf Baumwolle werden damit brillante blaue Färbungen mit guten Echtheiten erhalten.

Beispiel 179

12,2 g 1-Amino-4-(3'-amino-4'-hydroxysulfonylphenylamino)-2-hydroxysulfonylanthrachinon wurden in 250 ml Wasser eingerührt, wobei mit Natronlauge ein pH-Wert von 6,5 eingestellt wurde. In diese Lösung wurde bei 0 bis 5 °C eine Suspension aus 4,61 g Cyanurchlorid in 100 g Eiswasser eingetragen. Unter Aufrechterhaltung von pH 6,5 wurde bei 0 bis 5 °C gerührt bis die Umsetzung beendet war, was etwa 2 Stunden in Anspruch nahm.

Nach der Zugabe von 8,6 g 4-(2'-Chlorethylsulfonyl)-3-hydroxysulfonylmethylanilin wurde die Temperatur auf 35 °C erhöht und 2 Stunden bei dieser Temperatur gerührt.

Nach dem Abkühlen auf Raumtemperatur wurde mit 100 g Kochsalz ausgesalzen, der ausgefallene Farbstoff abgesaugt und getrocknet.

Er färbt Baumwolle in blauem Ton mit guten Echtheiten und entspricht der Formel

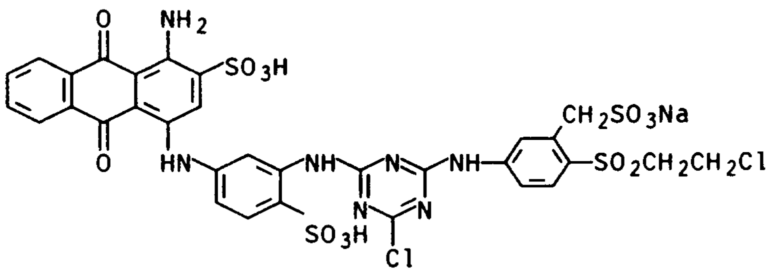

Beispiel 180

Einen weiteren Farbstoff mit ähnlichen Eigenschaften erhielt man, als man anstelle von 1-Amino-4-(3'-amino-4-hydroxysulfonylphenylamino)-2-hydroxysulfonylanthrachinon 13,2 g 1-Amino-4-(3'-amino-5'-hydroxysulfonyl-2',4',6'-trimethylphenylamino)-2-hydroxysulfonylanthrachinon verwendete.

Er entspricht der Formel

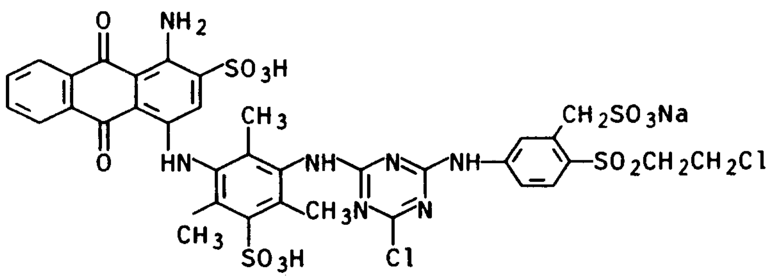

In analoger Weise werden die in der folgenden Tabelle 11 aufgeführten Verbindungen der Formel

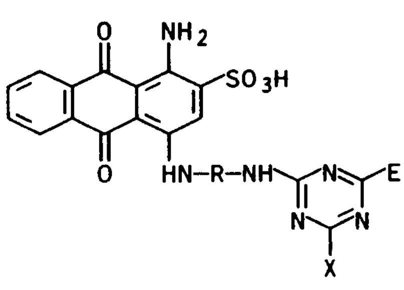

erhalten.

Tabelle 11

| Bsp.-Nr. | R | X | E | Farbton auf Baumwolle |
|---|---|---|---|---|
| 181 | 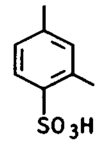 | Cl | E-2 | |
| 182 | 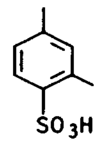 | F | E-2 | |
| 183 | 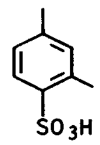 | F | E-1 | |
| 184 | 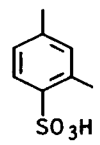 | Cl | E-3 | |
| 185 | 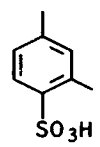 | F | E-3 | |
| 186 | 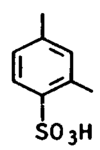 | Cl | E-4 | |
| 187 | 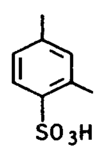 | F | E-4 | |
| 188 | 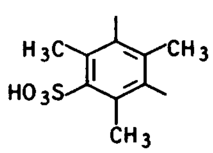 | F | E-1 | |
| 189 | 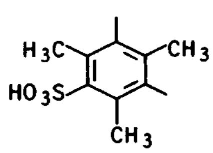 | Cl | E-2 | |

Fortsetzung - Tabelle 11

| Bsp.-Nr. | R | X | E | Farbton auf Baumwolle |
|---|---|---|---|---|
| 190 | 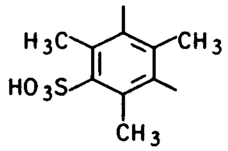 | F | E-2 | |
| 191 | 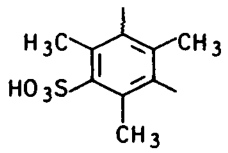 | Cl | E-3 | |
| 192 | 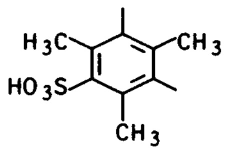 | F | E-3 | |
| 193 | 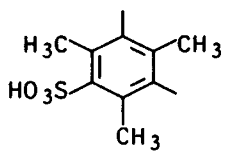 | Cl | E-4 | |
| 194 | 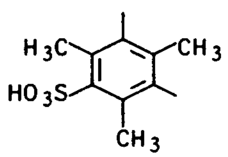 | F | E-4 | |

Beispiel 195

5 19,1 g der Verbindung der Formel

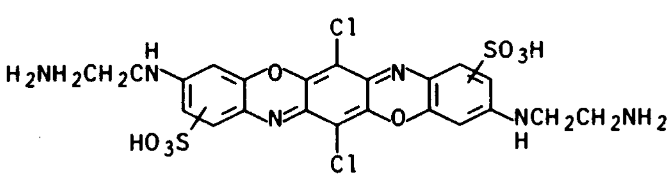

wurden in 1000 g Wasser eingerührt, wobei mit Natronlauge ein pH-Wert von 10 eingestellt wurde. Diese Lösung wurde in eine 40 bis 50 °C warme, auf pH 6 bis 8 gestellte Lösung des Kondensationsproduktes aus 11,1 g Cyanurchlorid mit 18,8 g 4-(2'-Chlorethylsulfonyl)-3-hydroxysulfonylmethylanilin getropft. Unter Aufrechterhaltung eines pH-Wertes von 6,5 bis 7 wurde bei 60 °C gerührt bis die Umsetzung beendet war, was etwa 1 Stunde in Anspruch nahm. Nach dem Abkühlen auf Raumtemperatur wurde mit 500 g NaCl ausgesalzen, der ausgefallene Farbstoff abgesaugt und getrocknet. Er färbt Baumwolle in brillantem blauen Ton mit guten Echtheiten und entspricht der Formel

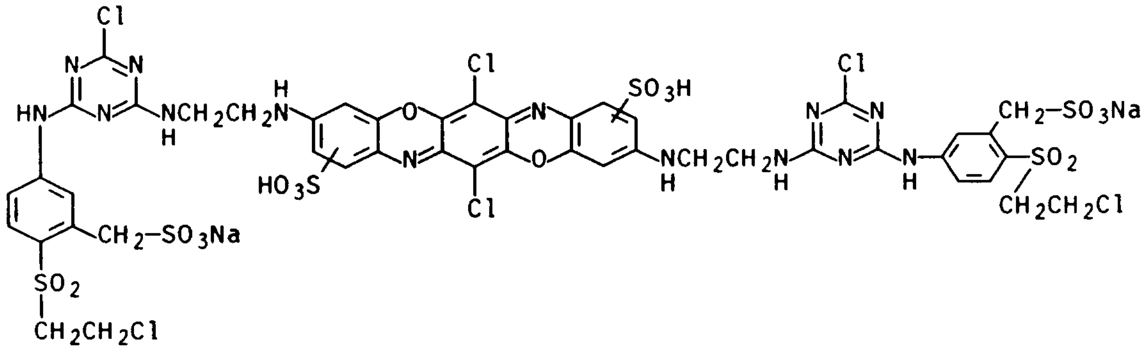

Weitere Farbstoffe, die in analoger Weise erhalten werden, sind in Tabelle 12 aufgeführt.

Tabelle 12

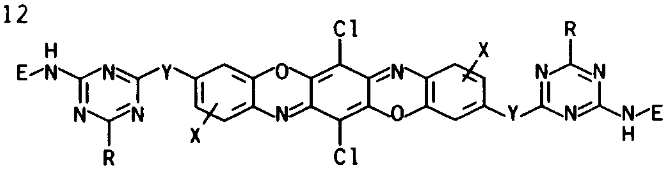

| Bsp.-Nr. | Y | X | R | E | Farbton auf Baumwolle |
|---|---|---|---|---|---|
| 196 | 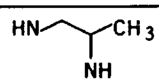 | $SO_3H$ | Cl | E-1 | blau |
| 197 | 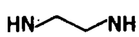 | $SO_3H$ | F | E-1 | blau |
| 198 | 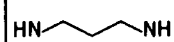 | $SO_3H$ | Cl | E-1 | blau |
| 199 | 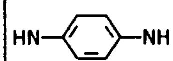 | $SO_3H$ | Cl | E-1 | blau |
| 200 | 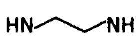 | $SO_2C_2H_4OSO_3H$ | Cl | E-1 | blau |
| 201 | 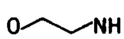 | $SO_3H$ | Cl | E-1 | rot |
| 202 | 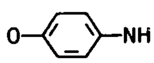 | $SO_3H$ | Cl | E-1 | rot |
| 203 | 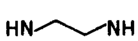 | $SO_3H$ | Cl | E-2 | blau |
| 204 | 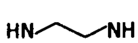 | $SO_3H$ | F | E-2 | blau |
| 205 | 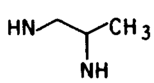 | $SO_3H$ | Cl | E-2 | blau |

Fortsetzung - Tabelle 12

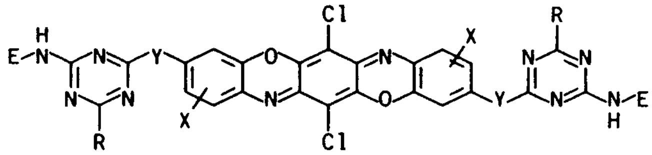

| Bsp.-Nr. | Y | X | R | E | Farbton auf Baumwolle |
|---|---|---|---|---|---|
| 206 | 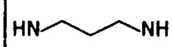 | $SO_3H$ | Cl | E-2 | blau |
| 207 | 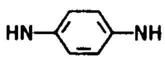 | $SO_3H$ | Cl | E-2 | blau |
| 208 | 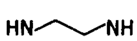 | $SO_2C_2H_4OSO_3H$ | Cl | E-2 | blau |
| 209 | 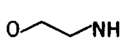 | $SO_3H$ | Cl | E-2 | rot |
| 210 | 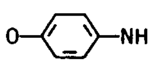 | $SO_3H$ | Cl | E-2 | rot |
| 211 | 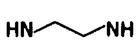 | $SO_3H$ | Cl | E-3 | blau |
| 212 | 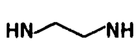 | $SO_3H$ | F | E-3 | blau |
| 213 | 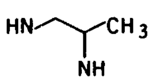 | $SO_3H$ | Cl | E-3 | blau |
| 214 | 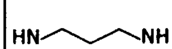 | $SO_3H$ | Cl | E-3 | blau |
| 215 | 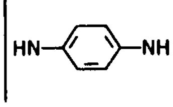 | $SO_3H$ | Cl | E-3 | blau |

Fortsetzung - Tabelle 12

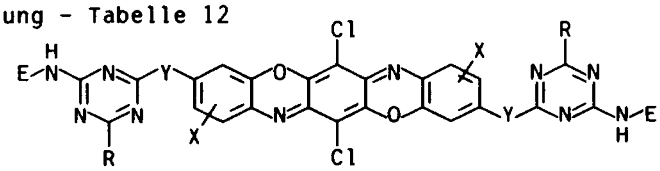

| Bsp.-Nr. | Y | X | R | E | Farbton auf Baumwolle |
|---|---|---|---|---|---|
| 216 | 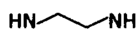 | $SO_2C_2H_4OSO_3H$ | Cl | E-3 | blau |
| 217 | 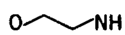 | $SO_3H$ | Cl | E-3 | rot |
| 218 | 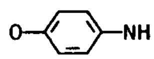 | $SO_3H$ | Cl | E-3 | rot |
| 219 | 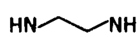 | $SO_3H$ | Cl | E-4 | blau |
| 220 | 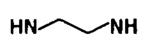 | $SO_3H$ | F | E-4 | blau |
| 221 | 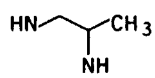 | $SO_3H$ | Cl | E-4 | blau |
| 222 | 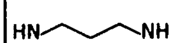 | $SO_3H$ | Cl | E-4 | blau |
| 223 | 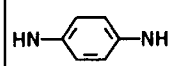 | $SO_3H$ | Cl | E-4 | blau |
| 224 | 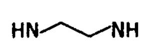 | $SO_2C_2H_4OSO_3H$ | Cl | E-4 | blau |
| 225 | 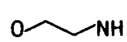 | $SO_3H$ | Cl | E-4 | rot |
| 226 | 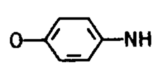 | $SO_3H$ | Cl | E-4 | rot |

Beispiel 227

Zu einer neutralen Lösung von 28,4 g 4-(2'-Chlorethylsulfonyl)-3-hydroxysulfonylmethylanilin in 200 ml Wasser gab man bei 5 bis 10 °C 97 g Kupferphthalocyanin-tetrasulfochlorid als feuchten Preßkuchen. Man

rührte 12 Stunden bei 20 bis 25 °C und hielt den pH-Wert durch Zugabe von 10%iger Sodalösung bei 6,5 bis 7,3. Danach wurde abgesaugt und der Filterrückstand getrocknet. Man isolierte 47 g des Farbstoffs der Formel

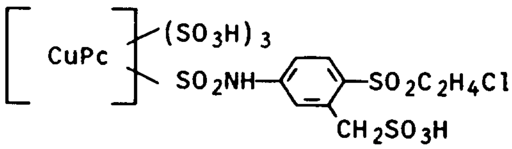

der 2,7 % NaCl enthielt. Der Farbstoff färbt Baumwolle in türkisem Farbton.

Beispiel 228

97 g Kupferphthalocyanin-tetrasulfochlorid wurden in 750 ml Wasser bei 0 bis 5 °C angerührt. Man gab 20,5 g Monoacetylethylendiamin zu und hielt den pH-Wert mit 10%iger Sodalösung bei 7,3 bis 7,5. Nach 12 Stunden bei 20 bis 25 °C wurde durch Zugabe von Natronlauge der pH-Wert auf 10 erhöht und eine Stunde bei 95 °C gerührt. Mit konz. Salzsäure wurde ein Niederschlag ausgefällt. Man saugte ab, wusch mit ca. 2 %iger Salzsäure nach und rührte in 500 ml Wasser an. Mit Natronlauge stellte man den pH-Wert der Lösung auf 7,0 bis 7,2 und gab 21,2 g Cyanurchlorid bei 0 bis 5 °C zu. Der pH-Wert wurde durch Zutropfen von Sodalösung bei 6,5 bis 7,0 gehalten. Nach 3 Stunden wurden 29 g 4-(2'-Chlorethylsulfonyl)-3-hydroxysulfonylmethylanilinzugegeben und die Temperatur auf 35 bis 40 °C erhöht. Die Farbstofflösung wurde spühgetrocknet. Man isolierte 185 g des Farbstoffs der Formel

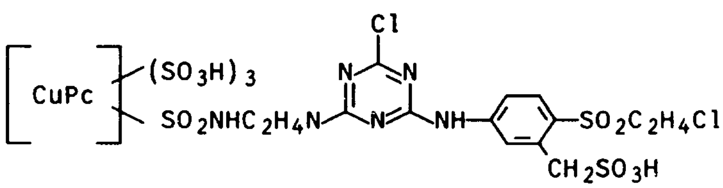

Der Farbstoff färbt Baumwolle in türkisem Farbton.

Beispiel 229

97 g Kupferphthalocyanin-tetrasulfochlorid wurden in 600 ml Wasser bei 0 bis 5 °C angerührt. Man gab 41 g N-Monoacetyl-m-phenylendiamin zu und hielt den pH-Wert durch Zugabe von 10 %iger Sodalösung bei 6,8 bis 7,0. Dann gab man 12 g 25 %ige Ammoniaklösung und 16 g Natriumacetat zu und erhöhte die Temperatur innerhalb 3 Stunden auf 50 °C und hielt den pH-Wert bei 7,0. Man ließ 250 g konz. Salzsäure zulaufen und erhöhte die Temperatur auf 90 bis 95 °C. Nach 4 Stunden wurde abgekühlt, der resultierende Niederschlag abgesaugt und neutral gewaschen. Ohne Trocknung wurde der Niederschlag in 750 ml Wasser angerührt und bei pH 7,0 und 0 bis 5 °C mit 19,4 g Cyanurchlorid versetzt. Der pH-Wert wurde durch Zugabe von Sodalösung bei 6,5 bis 7,0 gehalten. Nach 3 Stunden gab man 29 g 4-(2'-Chlorethylsulfonyl)-3-hydroxy-sulfonylmethylanilin zu und erhöhte die Temperatur auf 35 bis 40 °C. Die Farbstofflösung wurde sprühgetrocknet. Man isolierte 260 g des Farbstoffs der Formel

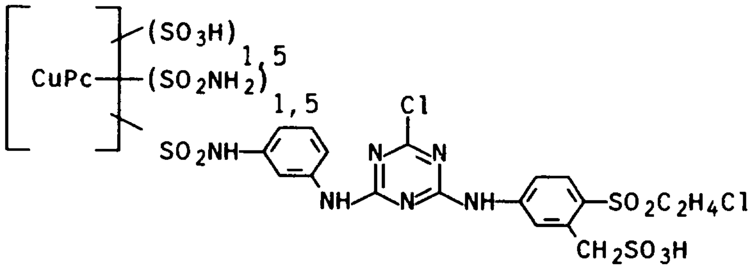

der Baumwolle in türkisem Farbton färbt.

Beispiel 230

768 g 2-Chlor-5-nitrobenzylchlorid und 470 g $Na_2SO_3$ wurden in 3,5 ml Wasser suspendiert. Es wurde auf 85 bis 90 °C erwärmt und 10 Stunden bei dieser Temperatur gerührt. Nach dem Abkühlen auf Raumtemperatur wurde das ausgefallene Produkt abfiltriert, mit wenig kaltem Wasser und Aceton gewaschen, anschließend unter vermindertem Druck bei 50 °C getrocknet.

Man erhielt 1815 g kristallines Produkt folgender Konstitution:

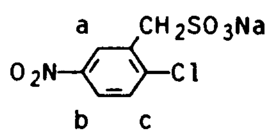

| | |
|---|---|
| Analyse: | ber. Chlor kovalent 12,98 |
| | gef. Chlor kovalent 12,22 |
| $^1$H-NMR: | 4,05 (s, $CH_2$), 7,70 (d, Arom.-$H^c$) |
| | 8,10 (dd, Arom.-$H^b$) |
| | 8,40 (d, Arom. $H^a$). |

Beispiel 231

360 g des in Beispiel 230 erhaltenen Produkts wurden in 750 ml N,N-Dimethylformamid gelöst und bei Raumtemperatur zunächst mit 182 g Pottasche und dann unter Eiskühlung mit 128,2 g 2-Mercaptoethanol versetzt.

Es wurde 2 Stunden bei 0 bis 5 °C nachgerührt anschließend auf 30 bis 35 °C erwärmt und weitere 16 Stunden gerührt.

Nach dem Abkühlen wurde das ausgefallene Produkt abfiltriert, mit Methanol gewaschen und unter vermindertem Druck bei 40 °C getrocknet. Man erhielt 490 g salzhaltiges Produkt folgender Konstitution:

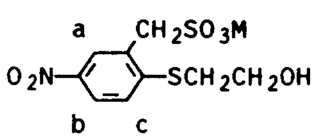

M = Na, K

| | |
|---|---|
| $^1$H-NMR: | 3,15 (t, $CH_2$), 3,60 (t, $CH_2$), 3,95 (s, $CH_2$) |
| | 5,10 (s, OH) |
| | 7,60 (d, Arom.-$H^c$) |
| | 8,08 (dd, Arom.-$H^b$) |
| | 8,30 (d, Arom.-$H^a$). |

Beispiel 232

550 g des in Beispiel 231 erhaltenen Produkts wurden bei Raumtemperatur in 1500 g konz. Salzsäure langsam eingetragen und dann auf 35 bis 40 °C erwärmt.

Bei dieser Temperatur wurden 255 g Chlor eingeleitet und bei 35 bis 40 °C nachgerührt bis die Umsetzung beendet war, was etwa 12 Stunden in Anspruch nahm.

Nach dem Abkühlen auf 10 °C wurde das ausgefallene Produkt abfiltriert, mit wenig 20%iger NaCl-Lösung gewaschen und unter vermindertem Druck bei 30 °C getrocknet.

Man erhielt 647 g salzhaltiges Produkt folgender Konstitution:

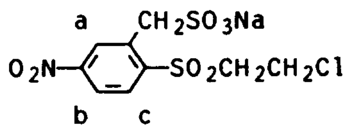

$^1$H-NMR: 3,82 (t, $CH_2$), 4,25 (t, $CH_2$), 4,55 (s, $CH_2$)
5,65 (s, $SO_3H$)
8,20 (d, Arom.-$H^c$)
8,30 (dd, Arom.-$H^b$)
8,60 (d, Arom.-$H^a$).

Beispiel 233

300 g der in Beispiel 232 erhaltenen Nitroverbindung wurden in 3000 g eines Methanol/Wasser-Gemisches (1:1 v/v) gelöst und bei Raumtemperatur in Gegenwart eines Palladiumkatalysators (10%ig auf Kohle) mit Wasserstoff hydriert.

Der Katalysator wurde abfiltriert und das Filtrat zur Trockene eingedampft. Es verblieben 152 g einer farblosen Verbindung, die der folgenden Struktur entspricht:

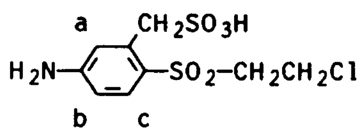

$^1$H-NMR: 3,65 (t, $CH_2$), 4,00 (t, $CH_2$), 4,20 (s, $CH_2$),
4,50 (br s, $NH_2$)
6,60 (dd, Arom.-$H^b$)
6,90 (d, Arom.-$H^a$)
7,50 (d, Arom.-$H^c$).

In analoger Weise werden die in Tabelle 13 aufgeführten Aniline der Formel

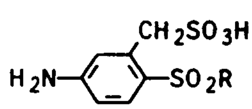

erhalten.

Tabelle 13

| Bsp.-Nr. | R |
|---|---|
| 238 | $CH=CH_2$ |
| 239 | $CH_2CH_2OH$ |
| 240 | $CH_2CH_2OSO_3H$ |
| 241 | $CH_2CH_2SSO_3H$ |

## Patentansprüche

**1.** Reaktivfarbstoffe der Formel I

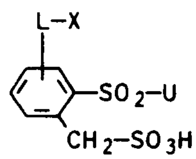

(I),

in der

U Vinyl, Propenyl oder den Rest der Formel

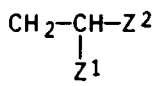

,

worin $Z^1$ für Wasserstoff oder Methyl und $Z^2$ für eine unter alkalischen Reaktionsbedingungen abspaltbare Gruppe stehen,

X im Fall a) den Rest eines Chromophors, der gegebenenfalls eine weitere reaktive Gruppe aufweist und der sich von einem gegebenenfalls metallisierten Mono- oder Disazofarbstoff, einem Triphendioxazin, einem Anthrachinon, einem Kupfer-Formazan oder einem metallisierten Phthalocyanin ableitet, oder
im Fall b) den Rest einer Kupplungskomponente, an den gegebenenfalls zusätzlich der Rest einer Diazokomponente über eine Azobrücke gebunden ist und der gegebenenfalls eine zusätzliche reaktive Gruppe aufweist, und

L im Fall a) ein Brückenglied der Formel

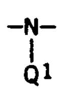 oder 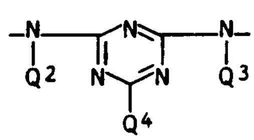 ,

worin $Q^1$ für Wasserstoff oder $C_1$-$C_4$-Alkyl, $Q^2$ und $Q^3$ gleich oder verschieden sind und unabhängig voneinander jeweils für Wasserstoff oder $C_1$-$C_4$-Alkyl und $Q^4$ für eine unter alkalischen Reaktionsbedingungen abspaltbare Gruppe stehen, oder im Fall b) eine Azobrücke bedeuten,

wobei der Rest X-L in meta- oder para-Stellung zum Rest $CH_2$-$SO_3H$ an den Benzolring geknüpft ist.

**2.** Reaktivfarbstoffe nach Anspruch 1, die der Formel Ia

X-L-$E^1$ (Ia)

entsprechen, in der X und L jeweils die in Anspruch 1 genannte Bedeutung besitzen und $E^1$ für einen Rest der Formel IIa

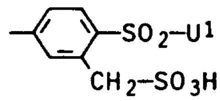

(IIa)

steht, in der

$U^1$ Vinyl oder den Rest der Formel $-CH_2-CH_2-Z^2$ bedeutet, wobei $Z^2$ die in Anspruch 1 genannte Bedeutung besitzt.

**3.** Benzylverbindungen der Formel III

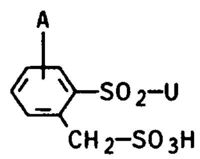

(III),

in der

A Nitro oder Amino und

U Vinyl, Propenyl oder den Rest der Formel

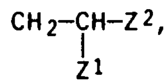

worin $Z^1$ für Wasserstoff oder Methyl und $Z^2$ für eine unter alkalischen Reaktionsbedingungen abspaltbare Gruppe stehen, bedeuten,

wobei der Rest A in meta- oder para-Stellung zum Rest $CH_2-SO_3H$ an den Benzolring geknüpft ist.

**4.** Verwendung der Reaktivfarbstoffe gemäß Anspruch 1 zum Färben oder Bedrucken von Hydroxygruppen oder Stickstoff enthaltenden Substraten.

## Claims

**1.** A reactive dye of the formula I

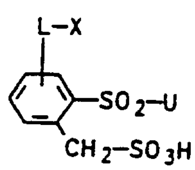

(I),

where

U is vinyl, propenyl or the radical of the formula

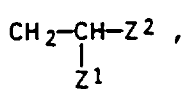

in which $Z^1$ is hydrogen or methyl and $Z^2$ is a group which is detachable under alkaline reaction conditions,

X is a) the radical of a chromophore which may contain a further reactive group and is derived from a metallized or unmetallized monoazo or disazo dye, from a triphendioxazine, from an anthraquinone, from a copper formazan or from a metallized phthalocyanine, or
b) the radical of a coupling component to which may be additionally attached the radical of a diazo component via an azo linkage and which may contain an additional reactive group, and

L is a) a bridge member of the formula

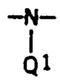 or 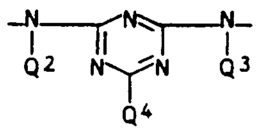 ,

where $Q^1$ is hydrogen or $C_1$-$C_4$-alkyl, $Q^2$ and $Q^3$ are identical or different and each is independently of the other hydrogen or $C_1$-$C_4$-alkyl, and $Q^4$ is a group which is detachable under alkaline reaction conditions, or
b) an azo linkage,

the radical X-L being bonded to the benzene ring meta or para to the $CH_2$-$SO_3H$ group.

**2.** A reactive dye as claimed in claim 1 which conforms to the formula Ia

X-L-$E^1$ (Ia)

where X and L are each as defined in claim 1 and $E^1$ is a radical of the formula IIa

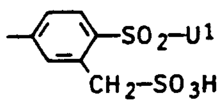 (IIa)

where

$U^1$ is vinyl or a radical of the formula -$CH_2$-$CH_2$-$Z^2$, in which $Z^2$ is as defined in claim 1.

**3.** A benzyl compound of the formula III

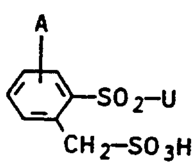 (III)

where

A is nitro or amino and

U is vinyl, propenyl or a radical of the formula

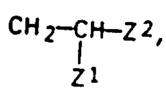

in which $Z^1$ is hydrogen or methyl and $Z^2$ is a group which is detachable under alkaline reaction conditions,

the radical A being bonded to the benzene ring meta or para to the $CH_2$-$SO_3H$ group.

**4.** The use of a reactive dye as claimed in claim 1 for dyeing or printing a hydroxyl- or nitrogen-containing substrate.

## Revendications

**1.** Colorants réactifs de formule I

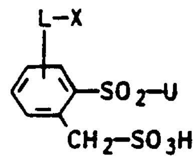

(I),

dans laquelle

U représente un groupe vinyle, propényle ou le reste de formule

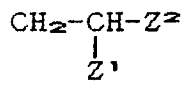

dans laquelle $Z^1$ est mis pour un atome d'hydrogène ou un groupe méthyle et $Z^2$ pour un groupe séparable deans des conditions réactionnelles alcalines,

X représente, dans le cas a), le reste d'un chromophore, qui présente éventuellement un autre groupe réactif et qui dérive d'un colorant mono- ou disazoïque éventuellement métallé, d'une triphénodioxazine, d'une anthraquinone, d'un cuivre-formazan ou d'une phtalocyanine métallée, ou

dans le cas b), le reste d'un composant de couplage, auquel est éventuellement lié de plus le reste d'un composant diazoïque par l'intermédiaire d'un pont azo, et qui présente éventuellement un groupe réactif supplémentaire, et

L représente, dans le cas a), un groupe pontant de formule

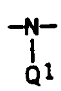

ou

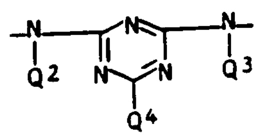

,

dans lequel $Q^1$ est mis pour un atome d'hydrogène ou un groupe alkyle en $C_1$-$C_4$, $Q^2$ et $Q^3$ sont identiques ou différents et représentent chacun indépendamment l'un de l'autre un atone d'hydrogène ou un groupe alkyle en $C_1$-$C_4$ et $Q^4$ est mis pour un groupe séparable dans des conditions réactionnelles alcalines, ou

dans le cas b), un pont azo,

dans lesquels le reste X-L est attaché au noyau benzène en position méta ou para par rapport au reste $CH_2$-$SO_3H$.

**2.** Colorants réactifs selon la revendication 1, qui correspondent à la formule Ia

X-L-$E^1$ (Ia)

dans laquelle X et L ont chacun la signification mentionnée dans la revendication 1 et $E^1$ est mis pour un reste de formule IIa

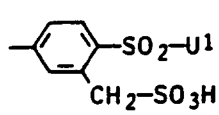

(IIa)

dans laquelle

$U^1$ représente un groupe vinyle ou le reste de formule $-CH_2CH_2Z^2$, dans laquelle $Z^2$ a la signification mentionnée dans la revendication 1.

**3.** Composés benzyliques de formule III

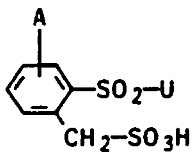

(III),

dans laquelle

A représente un groupe nitro ou amino et

U représente un groupe vinyle, propényle ou le reste de formule

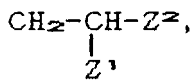

dans laquelle $Z^1$ est mis pour un atome d'hydrogène ou un groupe méthyle et $Z^2$ pour un groupe séparable dans des conditions réactionnelles alcalines,

le reste A étant attaché au noyau benzène en position méta ou para par rapport au reste $CH_2$-$SO_3H$.

**4.** Utilisation des colorants réactifs selon la revendication 1 pour teindre ou imprimer des substrats contenant des groupes hydroxy ou de l'azote.